(19) Europäisches Patentamt
European Patent Office
Office européen des brevets

(11) EP 0 445 796 B1

(12) **EUROPÄISCHE PATENTSCHRIFT**

(45) Veröffentlichungstag und Bekanntmachung des Hinweises auf die Patenterteilung:
**17.06.1998 Patentblatt 1998/25**

(21) Anmeldenummer: **91103462.7**

(22) Anmeldetag: **07.03.1991**

(51) Int Cl.[6]: **C07K 5/06**, C07K 5/08, A61K 7/02, C07C 257/18, C07D 295/185, A61K 31/195, A61K 31/395, C07D 211/62, C07D 211/60, C07D 207/04, C07D 207/16

(54) **Peptidbindungen enthaltende Essigsäurederivate**

Derivatives of acetic acid containing peptide bonds

Dérivés de l'acide acétique contenant des liaisons peptidiques

(84) Benannte Vertragsstaaten:
**AT BE CH DE DK ES FR GB GR IT LI LU NL SE**

(30) Priorität: **09.03.1990 CH 775/90**
**17.01.1991 CH 115/91**

(43) Veröffentlichungstag der Anmeldung:
**11.09.1991 Patentblatt 1991/37**

(73) Patentinhaber: **F. HOFFMANN-LA ROCHE AG**
**4002 Basel (CH)**

(72) Erfinder:
- **Alig, Leo, Dr.**
  **CH-4303 Kaiseraugst (CH)**
- **Edenhofer, Albrecht, Dr.**
  **CH-4125 Riehen (CH)**
- **Müller, Marcel, Dr.**
  **CH-4402 Frenkendorf (CH)**
- **Trzeciak, Arnold**
  **W-7860 Schopfheim (DE)**
- **Weller, Thomas, Dr.**
  **CH-4058 Basel (CH)**

(74) Vertreter: **Lederer, Franz, Dr. et al**
**Lederer, Keller & Riederer**
**Patentanwälte**
**Prinzregentenstrasse 16**
**80538 München (DE)**

(56) Entgegenhaltungen:
**EP-A- 0 183 271**

- **CHEMICAL ABSTRACTS, Band 103, 1985, Seite 418, Zusammenfassung Nr. 209699z, Columbus, Ohio, US; R. FERRONI et al.: "Ethyl esters of N-(3- and N-(4- amidinobenzoyl)(-L-)amino acids: synthesis and antiproteolytic activity towards bovine trypsin and porcine pancreatic kallikrein", & FARMACO ED. SCI. 1985, 40(10), 717-29**
- **CHEMICAL ABSTRACTS, Band 92, 1980, Seite 18, Zusammenfassung Nr. 208785s,**
- **Columbus, Ohio, US; D. LABES et al.: "Free-Wilson analysis of the inhibitory effect of 4-substituted benzamidines on thrombin, plasmin and trypsin", & PHARMAZIE 1979, 34(9), 554-6**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 445 796 B1

Printed by Jouve, 75001 PARIS (FR)

**Beschreibung**

Die vorliegende Erfindung betrifft neue Peptidbindungen enthaltende Essigsäurederivate, Verfahren zu deren Herstellung, pharmazeutische Präparate, die solche Verbindungen enthalten, sowie die Verwendung dieser Verbindungen bei der Herstellung pharmazeutischer Präparate.

Die Erfindung betrifft insbesondere Essigsäurederivate der Formel

$$H_2N(NH)C\text{-}X\text{-}Y\text{-}CO\text{-}Z\text{-}CH(Q^1)COOQ^2 \qquad (I)$$

worin

- $Q^1$ Wasserstoff, Methyl oder Phenyl,
- $Q^2$ Wasserstoff, Phenyl-nieder-alkyl oder unter physiologischen Bedingungen spaltbares nieder-Alkyl,
- X 1,4-Phenylen, 2,5- oder 3,6-Pyridylen oder über das C-Atom in 4-Stellung an die Gruppe Y gebundenes 1,4-Piperidinylen,
- Y eine Gruppe der Formel

$$-(CH_2)_{0\text{-}2}\text{-}CONHCH(Q^3)(CH_2)_{1\text{-}3}\text{-} \qquad (Y^1)$$

$$-CONHCH_2CH(Q^4)\text{-} \qquad (Y^2)$$

$$-(CH_2)_2NHCOCH_2\text{-} \qquad (Y^3)$$

$$-NHCO(CH_2)_3\text{-} \qquad (Y^4)$$

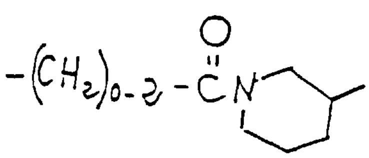

($Y^5$)

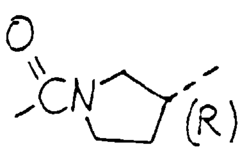

($Y^6$)

oder

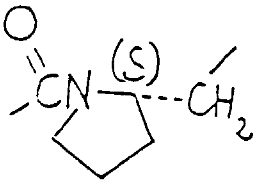

($Y^7$)

$Q^3$ Wasserstoff, Methyl, Phenyl, -COOH oder $-CONH(CH_2)_2-COOH$,
$Q^4$ Wasserstoff, Methyl oder Phenyl,
Z eine 1,4-Piperazinylengruppe, eine über das N-Atom in 1-Stellung an die CO-Gruppe gebundene 1,4-Piperidinylengruppe oder eine Gruppe der Formel

$$-NHCH(R^1)-$$

oder

$$-NHCH(COR^2)-$$

$R^1$ Wasserstoff, Methyl, Phenyl oder -COO-nieder-Alkyl,
$R^2$ der Rest einer über die Aminogruppe gebundenen α-Aminocarbonsäure oder eines Esters davon, oder eine Gruppe der Formel $-NHCH_2CH_2-Ar$, oder
$-CO-R^2$ eine Carbamoylgruppe oder eine Pyrrolidinoyl- oder Piperidinoylgruppe,
Ar Phenyl oder durch nieder-Alkyl, nieder-Alkoxy, -COOH, -COO-nieder-Alkyl, $O(CH_2)_{1-4}-COOH$ oder $O(CH_2)_{1-4}-COO$-nieder-Alkyl substituiertes Phenyl ist,

sowie Hydrate oder Solvate und physiologisch verwendbare Salze davon.

Wie die in CA92 (1980): 208 785s, CA 103 (1985): 209 699z und EP-A-0 183 271 beschriebenen Verbindungen enthalten zwar die vorliegenden Verbindungen der Formel I eine Amidinophenyl- oder Amidinobenzoylgruppe; sie haben jedoch eine andere Struktur als die vorbekannten Verbindungen.

Im Rahmen der vorliegenden Erfindung bezeichnet tBu t-Butyl, Boc t-Butoxycarbonyl, Z Benzyloxycarbonyl, Val L-Valyl, Phe L-Phenylalanyl, Ser L-Seryl, Gly Glycyl, Ala L-Alanyl, Asp L-α-Aspartyl, Leu L-Leucin, Tyr L-Tyrosin.

Der Ausdruck "nieder" bezeichnet Gruppen mit 1-6, vorzugsweise 1-4, C-Atomen. Beispiele von nieder-Alkylgruppen sind Methyl, Aethyl, Propyl, Isopropyl und n-, s- oder t-Butyl. Beispiele von unter physiologischen Bedingungen spaltbaren nieder-Alkylgruppen sind primäre und sekundäre nieder-Alkylgruppen.

Beispiele von über die Aminogruppe gebundenen α-Aminocarbonsäureresten sind Val, Phe, Ser, Leu, Tyr und deren entsprechenden nieder-Alkyl- oder Phenyl-niederalkyl-ester, Amide und Mono- oder Di-niederalkyl-amide.

Die Verbindungen der Formel I können solvatisiert, insbesondere hydratisiert sein. Die Hydratisierung kann im Zuge des Herstellungsverfahrens erfolgen oder allmählich als Folge hygroskopischer Eigenschaften einer zunächst wasserfreien Verbindung der Formel I auftreten.

Beispiele von physiologisch verwendbaren Salzen der Verbindungen der Formel I sind Salze mit physiologisch verträglichen Mineralsäuren, wie Salzsäure, Schwefelsäure oder Phosphorsäure, oder mit organischen Säuren, wie Methansulfonsäure, Essigsäure, Trifluoressigsäure, Zitronensäure, Fumarsäure, Bernsteinsäure oder Salicylsäure. Die Verbindungen der Formel I können auch Salze mit physiologisch verträglichen Basen bilden. Beispiele solcher Salze sind Alkalimetall-, Erdalkalimetall-, Ammonium- und Alkylammoniumsalze, wie das Na-, K-, Ca- oder Tetramethylammoniumsalz. Die Verbindungen der Formel I enthalten eine Amidinogruppe und können daher in Form von Zwitterionen vorliegen.

Die Verbindungen der Formel I, die ein oder mehrere asymmetrische C-Atome enthalten, können als Enantiomere, als Diastereomere oder als Gemische davon, z.B. als Racemate, vorliegen.

Bevorzugte Verbindungen der Formel I sind diejenigen der Formel

$$H_2N(HN)C-X-Y^a-CONHCH(R^{11})CH_2COOQ^{21} \qquad \text{I-A}$$

worin

X 1,4-Phenylen oder über das C-Atom in 4-Stellung an die Gruppe $Y^a$ gebundenes 1,4-Piperidinylen,
$Y^a$ eine Gruppe der Formel

$$-(CH_2)_{0-1}-CONHCH(Q^a)(CH_2)_{1-2}- \qquad (Y^{11})$$

$$-(CH_2)_2NHCOCH_2- \qquad (Y^3)$$

$-NHCO(CH_2)_3-$ ($Y^4$)

oder

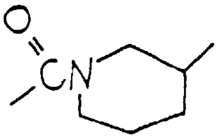

($Y^{51}$)

$Q^a$ Wasserstoff oder Phenyl,
$R^{11}$ Wasserstoff oder $-CO-R^{22}$,
$R^{22}$ der Rest einer über die Aminogruppe gebundenen α-Aminocarbonsäure oder eines Esters davon und
$Q^{21}$ Wasserstoff oder unter physiologischen Bedingungen spaltbares nieder-Alkyl ist.

In der Formel I ist Y vorzugsweise eine Gruppe der Formel $Y^1$, insbesondere

$-CONH(CH_2)_{2-4}-$,

$-CH_2CONH(CH_2)_2-$,

$-CONHCH(C_6H_5)CH_2-$,

$-CONHCH(CONHCH_2CH_2COOH)CH_2-$,

$-CONHCH(COOH)CH_2-$

oder

$-CONHCH(CH_3)CH_2-$.

In der Formel I ist Z vorzugsweise eine Gruppe der Formel $-NHCH_2-$, $-NHCH(CH_3)-$, $-NHCH(C_6H_5)-$. -NHCH(COO-Isobutyl)-, -NHCH(CO-Val)-, -NHCH(CO-Phe)-, -NHCH(CO-Tyr)-, $-NHCH(CO\text{-}Ser\text{-}OC_2H_5)-$, -NHCH(CO-Leu-O-Isopropyl)-. $-NHCH(CONHCH_2CH_2-C_6H_4-OCH_3)-$, $-NHCH(CONHCH_2CH_2-C_6H_4-COOH)-$, $-NHCH(CONHCH_2CH_2-C_6H_4-OCH_2COOH)-$, $-NHCH(CONH_2)-$ oder -NHCH(Pyrrolidinoyl)- ist.

Besonders bevorzugte Verbindungen der Formel I sind folgende:

N-[N-[N-(p-Amidinobenzoyl)-β-alanyl]-L-α-aspartyl]-3-phenyl-L-alanin,
N-[N-[4-(p-Amidinobenzamido)butyryl]-L-α-aspartyl]-L-valin,
N-[N-(p-Amidinobenzoyl)-β-alanyl]-β-alanin,
N-[N-[N-(p-Amidinobenzoyl)-β-alanyl]-L-α-aspartyl]-L-leucinisopcopylester,
N-[N-[N-(p-Amidinobenzoyl)-β-alanyl]-L-α-aspartyl]-L-valin,
N-[N-[N-(p-Amidinobenzoyl)-β-alanyl]-L-α-aspartyl]-3-(p-hydroxyphenyl)-L-alanin,
N-[N-[5-(p-Amidinobenzamido)valeryl]-L-α-aspartyl]-3-phenyl-L-alanin,
N-[5-(p-Amidinobenzamido)valeryl]-L-α-asparaginsäure-isobutylester,
N-[N-[N-(p-Amidinobenzoyl)-β-alanyl]-L-α-aspartyl]-L-serinäthylester,
N-[N-[[(R)-1-(p-Amidinobenzoyl)-3-pyrrolidinyl]carbonyl]-L-α-aspartyl]-3-phenyl-L-alanin und
N-[N-[N-[(5-Amidino-2-pyridyl)carbonyl]-β-alanyl]-L-α-aspartyl]-3-phenyl-L-alanin.

Weitere Beispiele von Verbindungen der Formel I sind folgende:

N-[N-[N-(1-Amidino-4-piperidinylcarbonyl)-β-alanyl]-L-α-aspartyl]-3-phenyl-L-alanin,
N-[N-[N-(p-Amidinophenylacetyl)-β-alanyl]-L-α-aspartyl]-3-phenyl-L-alanin,
N-[N-[4-(p-Amidinophenylcarbamoyl)butyryl]-L-α-aspartyl]-3-phenyl-L-alanin,
N-[N-[(p-Amidinophenylcarbamoyl)acetyl]-L-α-aspartyl]-3-phenyl-L-alanin,
rac-N-[1-(p-Amidinobenzoyl)-3-piperidinylcarbonyl]-β-alanin,
N-[4-(p-Amidinobenzamido)butyryl]-β-alanin
N-[(DL)-N-(p-Amidinobenzoyl)-3-phenyl-β-alanyl]-β-alanin,
N,N'-[[(S)-(p-Amidinobenzamido)äthylen]dicarbonyl]di-β-alanin,
N2-(p-Amidinobenzoyl)-N4-(2-carboxyäthyl)-L-asparagin,
N-[5-(p-Amidinobenzamido)valeryl]-β-alanin,
rac-N-[[1-[3-(1-Amidino-4-piperidinyl)propionyl]-3-piperidinyl]carbonyl]-β-alanin,
N-[[(S)-1-(p-Amidinobenzoyl)-2-pyrrolidinyl]acetyl]-β-alanin,
(S)-3-[[N-(p-Amidinobenzoyl)-β-alanyl]amino-3-[(p-methoxyphenäthyl)carbamoyl]propionsäure,
N-[[(R)-1-(p-Amidinobenzoyl)-3-pyrrolidinyl]carbonyl]-β-alanin,
N-[N-(p-Amidinobenzoyl)-2-methyl-β-alanyl]-L-asparaginsäure-α-amid,
N-[N-[(p-Amidinophenyl)acetyl]-β-alanyl]-β-alanin,
rac-N-[[[1-(p-Amidinophenyl)acetyl]-3-piperidinyl]carbonyl]-β-alaninbenzylester,
rac-N-[[1-[(p-Amidinophenyl)acetyl]-3-piperidinyl]carbonyl]-β-alanin,
N-[N-[N-[3-(1-Amidino-4-piperidinyl)propionyl]-β-alanyl]L-α-aspartyl]-3-phenyl-L-alanin,
(S)-β-[[DL-N-(p-Amidinobenzoyl)-3-methyl-β-alanyl]amino]-γ-oxo-1-pyrrolidinbuttersäure,
DL-N-[N-(p-Amidinobenzoyl)-β-alanyl]-3-methyl-β-alanin,
N-[DL-N-(p-Amidinobenzoyl)-2-phenyl-β-alanyl]-β-alanin,
DL-N-[N-(p-Amidinobenzoyl)-β-alanyl]-2-methyl-β-alanin,
DL-N-[N-(p-Amidinobenzoyl)-β-alanyl]-2-phenyl-β-alanin,
p-[2-[[N-[N-(p-Amidinobenzoyl)-β-alanyl]-L-α-aspartyl]-amino]äthyl]benzoesäure,
DL-N-[N-(p-Amidinobenzoyl)-β-alanyl-3-phenyl-β-alanin,
[p-[2-[[N-[N-(p-Amidinobenzoyl)-β-alanyl]-L-α-aspartyl]amino]äthyl]phenoxy]essigsäure,
1-[N-(p-Amidinobenzoyl)-β-alanyl]-4-piperidinessigsäure,
4-[N-(p-Amidinobenzoyl)-β-alanyl]-1-piperazinessigsäure.

Die obigen Verbindungen können erfindungsgemäss dadurch erhalten werden, dass man aus einer Verbindung der Formel

$$X'\text{-}Y\text{-}CO\text{-}Z'\text{-}CH(Q^1)\text{-}COO\text{-}Q^5 \qquad II$$

worin

| | |
|---|---|
| $Q^1$ und Y | die weiter oben angegebene Bedeutung haben, |
| X' | in 4-Stellung durch eine gegebenenfalls geschützte Amidinogruppe substituiertes Phenyl oder 4-Piperidinyl ist, und |
| Z' bzw. $Q^5$ | die gleiche Bedeutung wie weiter oben für Z bzw. $Q^2$ angegeben haben, |

zumindest eine Schutzgruppe abspaltet, oder
b) in einem Nitril der Formel

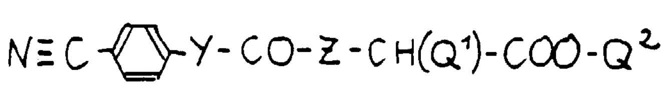

(III)

die Nitrilgruppe in die Amidinogruppe überführt, und gewünschtenfalls eine Verbindung der Formel I in ein physiologisch verträgliches Salz überführt oder ein Salz einer Verbindung der Formel I in die freie Säure oder Base überführt.

Beispiele von Schutzgruppen in den Verbindungen der Formel II sind Benzyl oder nieder-Alkylgruppen, wie t-Butyl, in den Estergruppen, wie Benzyl-OCO- bzw. t-Bu-OCO-, sowie Amidinoschutzgruppen, wie Z und Boc. Beispiele von geschützten Amidinogruppen sind -C(NH)NH-Z, -C(NH)NH-Boc und -C(N-Boc)-NH-Boc.

Estergruppen lassen sich in an sich bekannter Weise abspalten, beispielsweise durch Verseifung mit einer Base,

wie einem Alkalimetallhydroxyd, z.B. Natriumhydroxid, in einem Lösungsmittel, wie Methanol. Benzylester können durch Hydrierung in Gegenwart eines Edelmetallkatalysators, wie Palladium auf Kohlenstoff (Pd/C) in einem Lösungsmittel, wie Methanol, Aethanol, Ameisensäure oder Essigsäure, bei einer Temperatur bis zu etwa 40°C, vorzugsweise bei Raumtemperatur gespalten werden. Dabei wird gleichzeitig eine in der Gruppe X' enthaltene Amidinoschutzgruppe, wie Z, abgespalten.

Estergruppen, wie t-Butyl, bzw. Amidinoschutzgruppen, wie Boc, lassen sich z.B. mit einer Säure, wie Ameisensäure oder Trifluoressigsäure, gewünschtenfalls in einem Lösungsmittel, wie Dichlormethan, bei einer Temperatur bis zu 40°C, vorzugsweise bei Raumtemperatur spalten.

Die Verbindungen der Formel II sind neu und ebenfalls Gegenstand der vorliegenden Erfindung. Ihre Herstellung kann ausgehend von bekannten Verbindungen nach an sich bekannten Methoden, z.B. wie nachstehend beschrieben, erfolgen.

So wird in einer ersten Stufe ein Amin der Formel

$$H\text{-}Z'\text{-}CH(Q^1)COO\text{-}Q^{22} \qquad IV$$

mit einer Säure der Formel

$$R^5\text{-}NHCH(Q^{31})(CH_2)_{1\text{-}3}\text{-}COOH \qquad Va$$

$$R^5\text{-}NHCH_2CH(Q^4)\text{-}COOH \qquad Vb$$

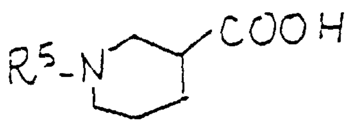

Vc

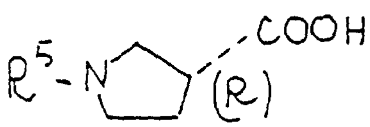

Vd

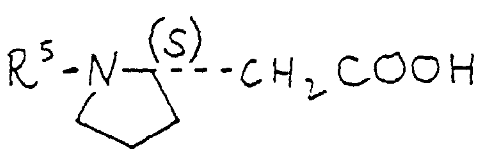

Ve

oder

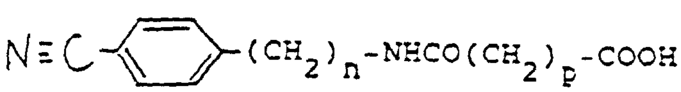

VI

worin

$Q^{22}$ eine leicht spaltbare Alkylgruppe,

$Q^{31}$ Wasserstoff, Methyl oder Phenyl,
$R^5$ eine Aminoschutzgruppe, wie Z oder Boc.

n = 0 und p = 3 oder n = 2 und p = 1 sind,
unter Bildung einer Amidgruppe nach aus der Peptidchemie an sich bekannten Methoden gekuppelt.

So kann man die Kupplung von IV mit Va, Vb oder VI z.B. in Tetrahydrofuran (THF) bei -10°C bis Raumtemperatur unter Argon in Gegenwart von O-Benzotriazol-1-yl-N,N,N',N' -tetramethyluroniumhexafluorphosphat (HBTU) durchführen. Die Kupplung von IV mit Vc, Vd oder Ve wird z.B. so bewerkstelligt, dass man zunächst die Säure Vc, Vd oder Ve in THF mit Chlordimethoxytriazin und N-Methylmorpholin aktiviert und dann mit dem p-Toluolsulfonat des Amins IV und N-Methylmorpholin umsetzt.

Aus dem so erhaltenen Reaktionsprodukt kann sodann eine darin enthaltene Aminoschutzgruppe $R^5$ selektiv abgespalten werden, z.B. Z oder Boc wie oben beschrieben durch katalytische Hydrierung bzw. mittels Trifluoressigsäure.

Ein auf diese Weise z.B. ausgehend von IV und Va erhaltenes Amin der Formel

$$H_2NCH(Q^{31})(CH_2)_{1-3}\text{-CO-Z'-}CH(Q^1)COO\text{-}Q^{22} \qquad \text{VIII}$$

kann man dann mit 4-Cyanbenzoesäure oder 4-Cyanphenylessigsäure zu einem Nitril, z.B. einem solchen der Formel III, kuppeln, z.B. wie oben für die Kupplung von IV mit Va beschrieben.

Ein so erhaltenes Nitril oder ein durch Kupplung von IV und VI erhaltenes Nitril, kann man z.B. durch Umsetzung mit Schwefelwasserstoff und Triäthylamin in Pyridin zum Thioamid, Methylierung mit Methyljodid in Aceton und anschliessende Umsetzung mit Ammoniumacetat in Methanol in eine Verbindung II überführen, in der X' eine freie Amidinogruppe enthält. Analog lässt sich ein Nitril III in die entsprechende Verbindung der Formel I überführen.

Ein Amin der obigen Formel VIII kann man auch z.B. mit 1-Amidino-4-piperidincarbonsäure, p-Amidinophenacetylchlorid oder p-Amidinobenzoylchlorid zu einer Verbindung II umsetzen, worin X' l-Amidino-4-piperidinyl bzw. p-Amidinophenyl ist.

Ein Amin der Formel VIII oder ein ausgehend von einer Verbindung der Formel IV und einer Säure einer der Formeln Va bis Ve erhaltenes Amin kann man ferner in Methylenchlorid und wässriger Natriumbicarbonatlösung mit p-Amidinobenzoylchlorid und anschliessend in Gegenwart von Natriumcarbonat mit Chlorameisensäurebenzylester bzw. mit Di-t-butyldicarbonat zu einer Verbindung der Formel II umsetzen, in der X' durch Z bzw. Boc geschütztes p-Amidinophenyl ist.

Die Verbindungen der Formeln IV-VI sind bekannt oder in Analogie zu den bekannten Verbindungen bzw. wie in den nachstehenden Beispielen beschrieben herstellbar.

So kann man ein Nitril VI durch Kupplung des entsprechenden Amins der Formel $NCC_6H_4(CH_2)_n\text{-}NH_2$ mit der entsprechenden Säure der Formel $HOOC(CH_2)_p\text{-}COOQ^{22}$, z.B. analog zur Kupplung eines Amins IV mit einer Säure V, gefolgt durch Abspaltung der Estergruppe $Q^{22}$ herstellen.

Zur Herstellung einer Verbindung der Formel II, worin X' eine in 4-Stellung durch eine geschützte Amidinogruppe substituiertes 4-Piperidinyl ist, kann man zunächst eine Verbindung der Formel

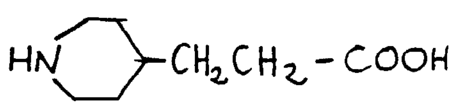

IX

in t-Butanol und Natronlauge z.B. mit N,N'-Bis(t-butoxycarbonyl)-S-methylisothioharnstoff zu einer Verbindung der Formel

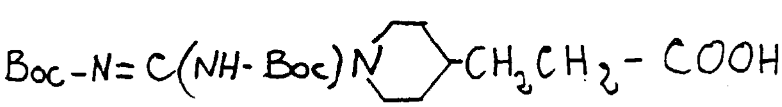

X

umsetzen und letztere dann z.B. mit einer Verbindung der Formel

$H_2NCH(Q^{31})(CH_2)_{1\text{-}3}\text{-}CONHCH_2CH_2COOCH_2C_6H_5$ XIa

$H_2NCH_2CH(Q^4)CONHCH_2CH_2COOCH_2C_6H_5$ XIb

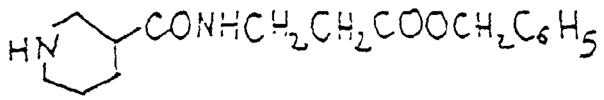

XIc

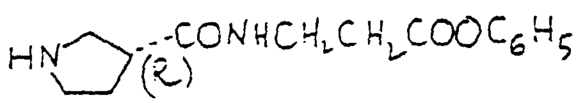

XId

oder

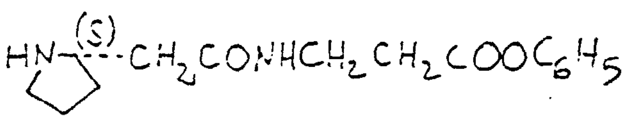

XIe

worin

$Q^{31}$ Wasserstoff, Methyl oder Phenyl ist,

koppeln.

Eine Verbindung der Formel II, worin X' eine in 4-Stellung durch eine geschützte Amidinogruppe substituiertes Phenyl oder 4-Piperidinyl ist, lässt sich ferner durch Kopplung einer Säure der Formel X'-Y-COOH mit einem Amin der obigen Formel IV herstellen.

Die Verbindungen der Formel I, ihre Solvate und ihre Salze hemmen sowohl die Bindung von Fibrinogen, Fibronectin und des Willebrand-Faktors an den Fibrinogenrezeptor der Blutplättchen (Glykoprotein IIb/IIIa) als auch die Bindung derselben und weiterer adhäsiver Proteine, wie Vitronectin, Kollagen und Laminin, an die entsprechenden Rezeptoren auf der Oberfläche verschiedener Zelltypen. Die besagten Verbindungen beeinflussen somit Zell-Zell- und Zell-Matrix--Wechselwirkungen. Sie verhindern insbesondere die Entstehung von Blutplättchenthromben und können bei der Bekämpfung bzw. Verhütung von Krankheiten wie Thrombose, Hirnschlag, Herzinfarkt, Entzündung und Arteriosklerose verwendet werden. Ferner haben diese Verbindungen einen Effekt auf Tumorzellen, indem sie deren Metastasierung hemmen. Somit können sie auch als Antitumor-Mittel eingesetzt werden. Weiter können sie die Wundheilung beschleunigen.

Die Hemmung der Fibrinogenbindung an den Fibrinogenrezeptor, Glykoprotein IIb/IIIa, kann wie folgt nachgewiesen werden:

Das Glykoprotein IIb/IIIa wird aus Triton X-100 Extrakten von menschlichen Blutplättchen gewonnen und durch Lectin-Affinitätschromatographie (Analytical Biochemistry 151, 1985, 169-177) und Chromatographie an einer Arg-Gly-Asp-Ser-Affinitätssäule (Science 231, 1986, 1559-62) gereinigt. Das so erhaltene Rezeptorprotein wird an Mikrotiterplatten gebunden. Die spezifische Bindung von Fibrinogen an den immobilisierten Rezeptor wird mit Hilfe eines ELISA-Systems ("enzyme-linked immunosorbent assay") bei stimmt. Die nachstehenden $IC_{50}$-Werte entsprechen derjenigen Konzentration der Testsubstanz, welche benötigt wird, um die Bindung von Fibrinogen an den immobilisierten Rezeptor um 50% zu hemmen:

| Produkt von Beispiel: | 1 | 2 | 3 | 4 | 5 | 6 | 7 |
|---|---|---|---|---|---|---|---|
| $IC_{50}$ (µM) | 0,0003 | 0,13 | 0,045 | 0,035 | 0,01 | 0,01 | 0,027 |

| Produkt von Beispiel: | 8 | 9 | 10 | 11 | 18 | 25 | 28 | 31 | 38 | 39 |
|---|---|---|---|---|---|---|---|---|---|---|
| $IC_{50}$ (µM) | 0,10 | 0,005 | 0,022 | 0,0025 | 0,010 | 0,0067 | 0,005 | 0,0128 | 0,0036 | 0,0012 |

Wie eingangs erwähnt, sind Arzneimittel, enthaltend eine Verbindung der Formel I, ein Solvat davon oder ein Salz davon, ebenfalls Gegenstand der vorliegenden Erfindung, weiterhin auch ein Verfahren zur Herstellung derartiger Arzneimittel, welches dadurch gekennzeichnet ist, dass man eine oder mehrere der besagten Verbindungen und erwünschtenfalls einen oder mehrere andere therapeutisch wertvolle Stoffe in eine galenische Darreichungsform bringt. Die Arzneimittel können enteral, z.B. oral in Form von Tabletten, Lacktabletten, Dragees, Hart- und Weichgelatinekapseln, Lösungen, Emulsionen oder Suspensionen, oder rektal, z.B. in Form von Suppositorien, oder als Spray verabreicht werden. Die Verabreichung kann aber auch parenteral, z.B. in Form von Injektionslösungen oder als Infusion, erfolgen.

Zur Herstellung von Tabletten, Lacktabletten, Dragees und Hartgelatinekapseln kann der Wirkstoff mit pharmazeutisch inerten, anorganischen oder organischen Excipientien vermischt werden. Als solche Excipientien kann man für Tabletten, Dragees und Hartgelatinekapseln z.B. Lactose, Maisstärke oder Derivate davon, Talk, Stearinsäure oder deren Salze, verwenden. Für Weichgelatinekapseln eignen sich als Excipientien z.B. vegetabile Oele, Wachse, Fette, halbfeste und flüssige Polyole; je nach Beschaffenheit des Wirkstoffes sind jedoch bei Weichgelatinekapseln überhaupt keine Excipientien erforderlich. Zur Herstellung von Lösungen und Sirupen eignen sich als Excipientien z.B. Wasser, Polyole, Saccharose, Invertzucker und Glukose, für Injektionslösungen eignen sich z.B. Wasser, Alkohole, Polyole, Glycerin und vegetabile Oele, und für Suppositorien eignen sich z.B. natürliche oder gehärtete Oele, Wachse, Fette und halbflüssige oder flüssige Polyole. Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten.

Für die Bekämpfung bzw. Verhütung der weiter oben genannten Krankheiten kann die Dosierung des Wirkstoffes innerhalb weiter Grenzen variieren und ist natürlich in jedem einzelnen Fall den individuellen Gegebenheiten anzupassen. Im allgemeinen dürfte bei oraler Verabreichung eine Dosis von etwa 0,1 bis 20 mg/kg, vorzugsweise von etwa 0,5 bis 4 mg/kg, pro Tag für den Erwachsenen angemessen sein, wobei aber die soeben angegebene obere Grenze auch überschritten werden kann, wenn sich dies als angezeigt erweisen sollte.

Beispiel 1

Zu einer Suspension von 100 mg N-[N-[N-(p-Amidinobenzoyl)-β-alanyl]-3-(t-butoxycarbonyl)-L-alanyl]-3-phenyl-L-alanin-t-butylester-hydrojodid in 10 ml Dichlormethan gibt man 15 ml Trifluoressigsäure. Nach 3 Stunden bei Raumtemperatur werden die Lösungsmittel abgedampft und der Rückstand mit Aether zur Kristallisation gebracht. Nach

Umkristallisation aus Methanol/Essigester erhält man 32 mg des Trifluoracetats von N-[N-[N-(p-Amidinobenzoyl)-β-alanyl]-L-α-aspartyl]-3-phenyl-L-alanin, Smp. 216-220°C (Zersetzung).

Das Ausgangsmaterial kann man wie folgt herstellen:

a) Zu einer Lösung von 446 mg Z-β-Ala-OH und 785 mg H-Asp(O-tBu)-Phe-O-tBu (gewonnen aus der Kondensation von Z-Asp(O-tBu)-OH mit H-Phe-O-tBu gefolgt von Hydrogenolyse) in 15 ml THF gibt man unter Argon bei 0°C 834 mg HBTU und 0,24 ml N-Methylmorpholin. Nach 5 Stunden wird eingeengt und der Rückstand auf Essigester/5% $NaHCO_3$ verteilt. Man wäscht mit Wasser und 1M $KHSO_4$. Die organischen Extrakte werden getrocknet, filtriert und eingeengt. Der Rückstand wird aus Essigester/Diisopropyläther umkristallisiert, wobei man 592 mg Z-β-Ala-Asp(O-tBu)-Phe-O-tBu, Smp. 138-139°C erhält.

b) Durch katalytische Hydrierung der Vorstufe (550 mg) in Aethanol in Gegenwart von 10% Pd/C erhält man nach Chromatographie an Kieselgel mit Essigester/Methanol 4:1→1:1 417 mg H-β-Ala-Asp(O-tBu)-Phe-O-tBu, MS: 464 $(M+H)^+$.

c) Durch Kupplung von 350 mg des Produkts von b) mit 125 mg 4-Cyanobenzoesäure auf die in Beispiel 1a) bechriebene Weise isoliert man nach Chromatographie an Kieselgel mit Essigester 293 mg N-[3-(t-Butoxycarbonyl)-N-[N-(p-cyanobenzoyl)-β-alanyl]-L-alanyl]-3-phenyl-L-alanin-t-butylester, Smp. 74-76°C.

d) Eine Lösung von 270 mg der Vorstufe in Pyridin/Triäthylamin 15:1 wird mit Schwefelwasserstoff gesättigt. Nach 24 Stunden werden die Lösungsmittel entfernt und der Rückstand auf Essigester/5% $NaHCO_3$ verteilt. Die organischen Extrakte werden mit Wasser und mit 1M Kaliumhydrogensulfatlösung gewaschen, getrocknet und eingeengt. Nach Chromatographie des Rückstandes an Kieselgel mit Essigester gefolgt von Umkristallisation aus Hexan erhält man 230 mg N-[3-(t-Butoxycarbonyl)-N-[N-[p-thiocarbamoylbenzoyl] -β-alanyl]-L-alanyl]-3-phenyl-L-alanin-t-butylester, Smp. 101-103°C.

e) Zu einer Lösung von 200 mg der Vorstufe in 15 ml Aceton gibt man 2 ml Methyljodid. Nach 3 Stunden bei Siedetemperatur lässt man auf Raumtemperatur abkühlen und fällt das Produkt durch Zugabe von Diäthyläther aus. Man erhält 226 mg N-[3-(t-Butoxycarbonyl)-N-[N-[p-[1-(methylthio)formimidoyl]benzoyl]-β-alanyl]-L-alanyl]-3-phenyl-L-alanin--t-butylester-hydrojodid, Smp. 139-140°C.

f) Zu einer Lösung von 160 mg der Vorstufe in 10 ml Methanol gibt man 32 mg Ammoniumacetat. Das Reaktionsgemisch wird 4 Stunden bei Siedetemperatur gehalten. Nach Abkühlen auf Raumtemperatur wird die Lösung filtriert, eingeengt und mit Diäthyläther versetzt. Das ausgefallene Produkt wird abfiltriert und getrocknet. Man erhält 135 mg Hydrojodid von N-[N-[N-(p-Amidinobenzoyl)-β-alanyl]-3-(t-butoxycarbonyl) -L-alanyl]-3-phenyl-L-alanin-t-butylester, Smp. 162-163°C.

Beispiel 2

Aus 60 mg N-[N-[N-(1-Amidino-4-piperidinylcarbonyl)-β-alanyl]-3-(t-butoxycarbonyl)-L-alanyl]-3-phenyl-L-alanin-t-butylester erhält man durch Behandlung mit Trifluoressigsäure in Methylenchlorid wie in Beispiel 1 beschrieben nach Kristallisation aus Methanol/Diäthyläther 26 mg N-[N-[N-(1-Amidino-4-piperidinylcarbonyl)-β-alanyl] -L-α-aspartyl]-3-phenyl-L-alanin-trifluoracetat (2:3), Smp. 127-130°C.

Das Ausgangsmaterial wird wie folgt hergestellt:

Die Kupplung von 86 mg 1-Amidino-4-piperidincarbonsäure (Belg. Pat. 893.282, Nippon Chemiphar Co. Ltd.) mit 119 mg H-β-Ala-Asp(O-tBu)-Phe-O-tBu (Beispiel 1b) in Dioxan in Gegenwart von 58 mg Pyridiniumhydrochlorid auf die in Beispiel 1a) beschriebene Weise liefert nach Chromatographie an Kieselgel mit Methylenchlorid/Methanol 1:1 71 mg N-[N-[N-(1-Amidino-4-piperidinylcarbonyl)-β-alanyl]-3-(t-butoxycarbonyl)-L-alanyl]-3-phenyl-L-alanyl-t-butylester, MS: 617 $(M+H)^+$.

Beispiel 3

Aus 50 mg N-[N-[N-(p-Amidinophenylacetyl)-β-alanyl] -3-(t-butoxycarbonyl)-L-alanyl]-3-phenyl-L-alanin-t-butylester-hydrojodid erhält man durch Behandlung mit Trifluoressigsäure wie in Beispiel 1 beschrieben 36 mg des Trifluoracetats von N-[N-[N-(p-Amidinophenylacetyl)-β-alanyl]-L-α-aspartyl]-3-phenyl-L-alanin, Smp. 218-220°C (aus Diäthyläther).

Das Ausgangsmaterial kann man wie folgt herstellen:

a) Durch Kupplung von 142 mg 4-Cyanophenylessigsäure mit 371 mg H-β-Ala-Asp(O-tBu)-Phe-O-tBu (Beispiel 1b) erhält man auf die in Beispiel la) beschriebene Weise nach Chromatographie an Kieselgel mit Essigester/Methanol 250 mg N-[N-[N-(p-Cyanophenylacetyl)-β-alanyl]-3-t-butoxycarbonyl -L-alanyl]-3-phenyl-L-alanin-t-butylester, Smp. 93-94°C.

b) Die Thionierung von 230 mg der Vorstufe wie in Beispiel ld) beschrieben liefert nach Chromatographie an Kieselgel mit Essigester 160 mg N-[N-[N-(p-Thiocarbamoylphenylacetyl)-β-alanyl]-3-t-butoxycarbonyl-L-alanyl]-3-phenyl-L-alanin-t-butylester, MS: 607 $(M+H)^+$.

c) Die Methylierung von 120 mg der Vorstufe analog Beispiel 1e) ergibt nach Kristallisation 100 mg N-[N-[N-(p-Methylthioformimidoyl)phenyl)acetyl]-β-alanyl]-3-t-butoxycarbonyl-L-alanyl]-3-phenyl-L-alanin-t-butylester-hydrojodid, Smp. 103-104°C (Aceton/Diäthyläther).

d) Die Umsetzung von 90 mg der Vorstufe mit Ammoniumacetat wie in Beispiel 1f) beschrieben ergibt 62 mg N-[N-[N--(p-Amidinophenylacetyl)-β-alanyl]-3-t-butoxycarbonyl-L--alanyl]-3-phenyl-L-alanin-t-butylester-hydrojodid, Smp. 152°C (Methanol/Diäthyläther).

Beispiel 4

Durch Behandlung von 55 mg N-[N-[4-(p-Amidinophenylcarbamoyl)butyryl]-3-(t-butoxycarbonyl)-L-alanyl]-3-phenyl-L-alanin-t-butylester-hydrojodid mit Trifluoressigsäure wie in Beispiel 1 beschrieben erhält man nach Kristallisation aus Aethanol/Diäthyläther 31 mg N-[N-[4-(p-Amidinophenylcarbamoyl)butyryl]-L-α-aspartyl]-3-phenyl-L-alanin-trifluoracetat (5:4), Smp. 159-161°C.

Das Ausgangsmaterial kann man wie folgt herstellen:

a) Durch Kupplung von 1,18 g 4-Aminobenzonitril mit 1,25 ml Monomethylglutarat wie in Beispiel 1a) beschrieben erhält man nach Umkristallisation aus Essigester 1,83 g Methyl 4-[(p-cyanophenyl)carbamoyl]butyrat, Smp. 126-127°C.

b) Zu einer Lösung von 1 g der Vorstufe in 10 ml Methanol gibt man 6 ml 1N Natronlauge. Nach 7 Stunden wird das Reaktionsgemisch eingeengt, mit Essigester extrahiert und dann mit 1N Salzsäure neutralisiert. Der entstandene Niederschlag wird abfiltriert, mit Wasser gewaschen und getrocknet. Man erhält 575 mg 4-[(p-Cyanophenyl)carbamoyl]buttersäure, Smp. 200-201°C.

c) Durch Kupplung von 464 mg der Vorstufe mit 863 mg H-Asp(O-tBu)-Phe-O-tBu wie in Beispiel 1a) beschrieben erhält man nach Chromatographie an Kieselgel mit Essigester und anschliessendem Verrühren in Hexan 701 mg N-[3-(t-Butoxycarbonyl)-N-[4-(p-cyanophenylcarbamoyl)butyryl]-L-alanyl]-3-phenyl-L-alanin-t-butylester, Smp. 64-66°C.

d) Die Umsetzung von 350 mg der Vorstufe mit Schwefelwasserstoff in Analogie zu Beispiel 1d) liefert nach Umkristallisation aus Essigester 304 mg N-[3-(t-Butoxycarbonyl)-N-[4-(p-thiocarbamoylphenyl)butyryl]-L-alanyl]-3-phenyl-L-alanin-t-butylester, Smp. 178-179°C.

e) Die Methylierung von 150 mg der Vorstufe in Analogie zu Beispiel le) ergibt nach Kristallisation mit Diäthyläther 175 mg N-[3-(t-Butoxycarbonyl)-N-[4-[[p-[1-(methylthio)formimidoyl]phenyl]carbamoyl]butyryl]-L-alanyl]-3-phenyl-L-alanin-t-butylester-hydrojodid (1:1), Smp. 127-128°C.

f) Aus 90 mg der Vorstufe erhält man in Analogie zu Beispiel 1f) 60 mg N-[N-[4-(p-Amidinophenylcarbamoyl)butyryl]-3-(t-butoxycarbonyl)-L-alanyl]-3-phenyl-L-alanin -t-butylester-hydrojodid, Smp. 130-132°C.

Beispiel 5

Die Umsetzung von 100 mg N-[N-[(p-Amidinophenäthylcarbamoyl)acetyl]-3-(t-butoxycarbonyl)-L-alanyl]-3-phenyl-L-alanin-t-butylester-hydrojodid mit Trifluoressigsäure wie in Beispiel 1 beschrieben ergibt nach Kristallisation mit Diäthyläther 69 mg N-[N-[(p-Amidinophenäthylcarbamoyl)acetyl]-L-α-aspartyl]-3-phenyl-L-alanin-trifluoracetat, Smp. 141-143°C.

Das Ausgangsmaterial kann man wie folgt herstellen:

a) Aus 876 mg p-Cyanohydrozimtsäure (Pharmazie 28, 1973, 724) erhält man nach bekannter Reaktionssequenz (Organic Synthesis 51, 1971, 48) 370 mg p-(2-Aminoäthyl)benzonitril, MS: 147 $(M+H)^+$.

b) Zu einer Lösung von 731 mg p-(2-Aminoäthyl)benzonitril und 1,39 ml Triäthylamin in 10 ml THF tropft man bei-10°C eine Lösung von 0,59 ml Malonsäurechloridmethylester in 5 ml THF zu. Dann lässt man auf Raumtemperatur aufwärmen, giesst auf Eiswasser und stellt mit 1N Salzsäure auf pH 2. Das THF wird abgedampft und die wässrigen Extrakte mit Essigester extrahiert. Nach Trocknen und Einengen erhält man 380 mg Methyl-(p-cyanophenäthyl) malonamat, Smp. 125°C.

c) Analog Beispiel 4b) ergibt die Verseifung von 750 mg des Produkts von b) 365 mg N-(p-Cyanophenäthyl)malonaminsäure, Smp. 137-139°C.

d) Analog Beispiel 1a) gewinnt man durch Kupplung von 300 mg der Vorstufe mit 507 mg H-Asp(O-tBu)-Phe-O-tBu nach Chromatographie an Kieselgel mit Essigester 352 mg N-[3-(t-Butoxycarbonyl)-N-[(p-cyanophenäthylcarbamoyl)acetyl]-L-alanyl]-3-phenyl-L-alanin-t-butylester, MS: 607 $(M+H)^+$.

e) Analog Beispiel 1d), e) und f) liefert dieselbe Reaktionssequenz mit 380 mg des Produktes von d) nach Kristallisation mit Diisopropyläther 152 mg N-[N-[(p-Amidinophenäthylcarbamoyl)acetyl]-3-(t-butoxycarbonyl)-L-alanyl]-3-phenyl-L-alanin-t-butylester-hydrojodid, Smp. 91-93°C (Zersetzung).

Beispiel 6

Aus 200 mg N-[N-[4-(p-Amidinobenzamido)butyryl]-3-(t-butoxycarbonyl)-L-alanyl]-L-valin-t-butylester-hydrojodid erhält man in Analogie zu Beispiel 1 nach Umkristallisation aus Aethanol/Diäthyläther 119 mg des Trifluoracetats von N-[N-[4-(p-Amidinobenzamido)butyryl]-L-α-aspartyl]-L-valin, Smp. 174°C (Zersetzung).

Das Ausgangsmaterial kann man wie folgt herstellen:

a) Durch Kupplung von 783 mg Z-4-Aminobuttersäure mit 1 g H-Asp(O-tBu)-Val-O-tBu (gewonnen aus der Kondensation von Z-Asp(O-tBu)-OH mit H-Val-O-tBu gefolgt von Hydrogenolyse) wie in Beispiel la) beschrieben erhält man nach Chromatographie an Kieselgel mit Essigester und Kristallisation mit Hexan 1,17 g N-[N-[4-[1-(Benzyloxy) formamido]butyramido] -3-(t-butoxycarbonyl)-L-alanyl]-L-valin-t-butylester, Smp. 64-65°C.

b) Die Hydrogenolyse von 1,1 g des Produktes der Vorstufe analog zu Beispiel 1b) ergibt 1 g N-[N-(4-Aminobutyryl)-3-(t-butoxycarbonyl)-L-alanyl]-L-valin-t-butylester, Smp. 99-100°C.

c) Zu einer Lösung von 324 mg 4-Cyanbenzoesäure, 352 mg 2-Chlor-4,6-dimethoxy-1,3,5-triazin und 0,24 ml N-Methylmorpholin in 10 ml Dimethylformamid gibt man nach 2 Stunden bei 0°C eine Lösung von 859 mg des Produktes von b). Man lässt auf Raumtemperatur aufwärmen und erhält nach Aufarbeitung wie in Beispiel 1d) und nach Chromatographie an Kieselgel mit Essigester 772 mg N-[N-[4-(p-Cyanobenzamido)butyryl]-3-(t-butoxycarbonyl)-L-alanyl]-L-valin-t-butylester, MS: 559 $(M+H)^+$.

d) Unterwirft man 760 mg des Produktes von c) derselben Reaktionsfolge wie in den Beispielen 1d), e) und f) beschrieben, so erhält man nach Kristallisation mit Diisopropyläther 444 mg N-[N-[4-(p-Amidinobenzamido)butyryl]-3-(t-butoxycarbonyl)-L-alanyl-L-valin-t-butylester-hydrojodid, Smp. 105-107°C (Zersetzung).

Beispiel 7

1,09 g rac-N-1-[[[p-[N-(Benzyloxycarbonyl)amidino]benzoyl]-3-piperidinyl]carbonyl]-β-alaninbenzylester und 0,25 g Pd/C werden in 20 ml Essigsäure unter Wasserstoff gerührt. Der Katalysator wird abfiltriert und das Filtrat eingedampft. Der Rückstand wird in Wasser aufgenommen und eingedampft. Der Niederschlag wird in Methanol suspendiert, mit Ammoniak auf pH 8 eingestellt und gerührt, dann abgenutscht, mit Methanol gewaschen und getrocknet. Man erhält 530 mg rac-N-[[1-(p-Amidinobenzoyl)-3-piperidinyl]carbonyl]-β-alanin in Form des Hydrates (2:1), Smp. >265°C, MS: 347 (96, M+H).

Das Ausgangsmaterial kann man wie folgt herstellen:

a) rac-N-(t-Butoxycarbonyl)piperidin-3-carbonsäure (Can. J. Physiol. Pharmacol. 57, 1979, 763) wird in THF mit Chlordimethoxytriazin und N-Methylmorpholin aktiviert und dann mit β-Alaninbenzylester-p-toluolsulfonat (J. Org. Chem. 17, 1952, 1564) und N-Methylmorpholin zu rac-N-[[1-(t-Butoxycarbonyl)-3-piperidinyl]carbonyl]-β-alanin-

benzylester, Smp. 57-59°C, gekoppelt.

b) Durch Spaltung mit Trifluoressigsäure erhält man daraus das Trifluoracetat des rac-(3-Piperidinylcarbonyl)-β-alanin-benzylesters.

c) Dieses wird in Methylenchlorid/wässriger Natriumbicarbonatlösung mit p-Amidinobenzoylchlorid und anschliessend in Gegenwart von Natriumcarbonat mit Chlorameisensäurebenzylester zu rac-N-1-[[[p-[N-(Benzyloxycarbonyl)amidino]benzoyl] -3-piperidinyl]carbonyl]-β-alaninbenzylester umgesetzt, MS: 571 (10, M+H).

Beispiel 8

512 mg N-[4-[p-[N-(Benzyloxycarbonyl)amidino]benzamido]butyryl]-β-alaninbenzylester und 170 mg Pd/C werden in 10 ml Essigsäure unter Wasserstoff gerührt. Der Katalysator wird abfiltriert und das Filtrat eingedampft. Der Rückstand wird in Wasser gelöst und eingedampft. Der Rückstand wird in Wasser suspendiert, mit Ammoniak auf pH 7 eingestellt, abgenutscht, mit Wasser gewaschen und getrocknet. Man erhält 239 mg N-[4-(p-Amidinobenzamido)butyryl]-β-alanin, Smp. >250°C, MS: 321 (12, M+H).

Das Ausgangsmaterial kann man wie folgt herstellen:

a) 4-(t-Butoxycarbonylamino)buttersäure wird mit Chlordimethoxytriazin und N-Methylmorpholin in THF aktiviert und mit β-Alaninbenzylester-p-toluolsulfonat in Gegenwart von N-Methylmorpholin zu N-[4-(1-t-Butoxyformamido) butyryl] -β-alaninbenzylester, Smp. 54-55°C, umgesetzt.

b) In Trifluoressigsäure erhält man daraus das Trifluoracetat des N-(4-Aminobutyryl)-β-alaninbenzylesters.

c) Letzteres wird in Methylenchlorid/Wasser/Natriumhydrogencarbonat mit p-Amidinobenzoylchlorid und anschliessend in Gegenwart von Natriumcarbonat mit Chloraneisensäurebenzylester zu N-[4-[p-[N-(Benzyloxycarbonyl)amidino]benzamido]butyryl]-β-alaninbenzylester, Smp. 173-183°C, umgesetzt.

Beispiel 9

Analog Beispiel 8 erhält man aus N-[N-[p-[N-(Benzyloxycarbonyl)amidino]benzoyl]-β-alanyl]-β-alaninbenzylester das N-[N-(p-Amidinobenzoyl)-β-alanyl]-β-alanin, Smp. >250°C, MS: 307 (6, M+H), in Form des Hydrates (2:1).

Das Ausgangsmaterial kann man wie folgt herstellen:

a) N-(t-Butoxycarbonyl)-β-alanin und β-Alaninbenzylester werden analog obiger Beispiele zu N-[N-(t-Butoxycarbonyl)-β-alanyl]-β-alaninbenzylester gekoppelt, Smp. 84-85°C.

b) In Trifluoressigsäure erhält man daraus das Trifluoracetat des (β-Alanyl)-β-alaninbenzylesters.

c) Dieses wird mit p-Amidinobenzoylchlorid und anschliessend mit Chlorameisensäurebenzylester zu N-[N-[p-[N-(Benzyloxycarbonyl)amidino]benzoyl]-β-alanyl]-β-alaninbenzylester, Smp. 165-166°C, umgesetzt.

Beispiel 10

In Analogie zu Beispiel 8 erhält man aus N-[(DL)-N-[p-[N-(Benzyloxycarbonyl)amidino]benzoyl]-3-phenyl-β-alanyl]-β-alaninbenzylester das N-[(DL)-N-(p-Amidinobenzoyl)-3-phenyl-β-alanyl]-β-alanin als Hydrat (3:1), Smp. >250°C, MS: 383 (62, M+H).

Das Ausgangsmaterial kann man wie folgt herstellen:

a) DL-N-(t-Butoxycarbonyl)-3-phenyl-β-alanin und β-Alaninbenzylester werden analog obiger Beispiele zu N-[(DL)-N-(t-Butoxycarbonyl)-3-phenyl-β-alanyl]-β-alaninbenzylester, Smp. 143-144°C, gekoppelt.

b) In Trifluoressigsäure erhält man daraus das Trifluoracetat des [(DL)-3-Phenyl-β-alanyl]-β-alaninbenzylesters.

c) Dieses wird mit p-Amidinobenzoylchlorid und anschliessend mit Chlorameisensäurebenzylester zu N-[(DL)-N-[p-[N-(Benzyloxycarbonyl)amidino]benzoyl]-3-phenyl-β-alanyl]-β-alaninbenzylester, Smp. 187-189°C, umgesetzt.

Beispiel 11

443 mg N-[3-[(Benzyloxy)carbonyl]-N-[5-[p-[N-[(benzyloxy)carbonyl]amidino]benzamido]valeryl]-L-alanyl]-3-phenyl-L-alaninbenzylester und 111 mg Pd/C werden in 9 ml Essigsäure 3 1/2 Stunden unter Wasserstoffgas gerührt. Die Lösung wird filtriert, eingedampft, der Rückstand in Wasser gelöst und die Lösung wieder eingedampft. Der Rückstand wird in Wasser verrührt, abgenutscht und getrocknet. Man erhält 246 mg N-[N-[5-(p-Amidinobenzamido)valeryl]-L-α-aspartyl]-3-phenyl-L-alanin, Smp. 241°C, als Hydrat (1:2).

Der Ausgangsester, Smp. 169-171°C, kann wie folgt hergestellt werden:

a) N-(t-Butoxycarbonyl)-L-asparaginsäure-4-benzylester wird mit 3-Phenyl-L-alaninbenzylester zu N-[3-[(Benzyloxy)carbonyl]-N-(t-butoxycarbonyl)-L-alanyl]-3-phenyl-L-alaninbenzylester, Smp. 93-94°C, gekoppelt.

b) Dieser wird mit Trifluoressigsäure entschützt und mit 5-(1-t-Butoxyformamido)valeriansäure zu N-[3-[(Benzyloxy)carbonyl]-N-[5-(1-t-butoxyformamido)valeryl]-L-alanyl]-3-phenyl-L-alaninbenzylester, Smp. 119,5-120,5°C, gekoppelt.

c) Letzterer wird in Trifluoressigsäure von der t-Butoxycarbonylschutzgruppe befreit und danach in Methylenchlorid/Wasser/Natriumbicarbonat mit p-Amidinobenzoylchlorid und anschliessend mit Chlorameisensäurebenzylester ins Ausgangsmaterial übergeführt.

Beispiel 12

Analog Beispiel 11 erhält man aus N,N'-[[(S)-[p-[N-[(Benzyloxy)carbonyl]amidino]benzamido]äthylen]dicarbonyl]-di-β-alanindibenzyldiester nach Abdampfen mit Wasser und Verrühren mit Methanol das N,N'-[[(S)-(p-Amidinobenzamido)äthylen]dicarbonyl]di-β-alanin, Smp. >250°C.

Den Ausgangsester, Smp. 171-172°C, erhält man wie folgt:

a) N-(t-Butoxycarbonyl)-L-asparaginsäure wird mit zwei Aequivalenten β-Alaninbenzylester zum N,N'-[[(S)-(1-t-Butoxyformamido)äthylen]dicarbonyl]di-β-alanindibenzylester, Smp. 109-110°C, gekoppelt.

b) Nach Spaltung der t-Butoxycarbonylgruppe mit Trifluoressigsäure setzt man in Methylenchlorid/Wasser/Natriumbicarbonat mit p-Amidinobenzoylchlorid und anschliessend mit Chlorameisensäurebenzylester zum Ausgangsester um.

Beispiel 13

Analog Beispiel 11 erhält man aus N-[(S)-N-[p-[N-[(Benzyloxy)carbonyl]amidino]benzoyl]-3-[(benzyloxy)carbonyl]-β-alanyl]-β-alaninbenzylester das N2-(p-Amidinobenzoyl)-N4-(2-carboxyäthyl)-L-asparagin, Smp. 212°C (Zers.).

Den Ausgangsester erhält man wie folgt:

a) N-(t-Butoxycarbonyl)-L-asparaginsäure-1-benzylester wird mit β-Alaninbenzylester zum 3-[[2-[(Benzyloxy)carbonyl]äthyl]carbamoyl]-N-(t-butoxycarbonyl)-L-alaninbenzylester, Smp. 77-78°C, gekoppelt.

b) Nach Spaltung der t-Butoxycarbonylschutzgruppe in Trifluoressigsäure koppelt man in Methylenchlorid/Wasser/Natriumbicarbonat mit p-Amidinobenzoylchlorid und setzt anschliessend mit Chlorameisensäurebenzylester zum Ausgangsester, Smp. 122-123°C, um.

Beispiel 14

Analog Beispiel 8 erhält man aus dem N-[5-[p-[N-[(Benzyloxy)carbonyl]amidino]benzamido]valeryl]-β-alaninbenzylester das N-[5-(p-Amidinobenzamido)valeryl]-β-alanin, Smp. >280°C.

Der Ausgangsester kann wie folgt hergestellt werden:

a) 5-(1-t-Butoxyformamido)valeriansäure wird mit 13-Alaninbenzylester zum N-[5-(1-t-Butoxyformamido)valeryl]-β-alaninbenzylester, Smp. 69-70°C, gekoppelt.

b) In Trifluoressigsäure erhält man daraus das Trifluoracetat des N-(5-Aminovaleryl)-β-alaninbenzylesters.

c) Dieses wird in Methylenchlorid/Wasser in Gegenwart von Natriumbicarbonat mit p-Amidinobenzoylchlorid und anschliessend mit Chlorameisensäurebenzylester zum Ausgangsester, Smp. 161-161,5°C, umgesetzt.

Beispiel 15

704 mg rac-N-[[1-[3-[1-[(E/Z))-N,N'-Bis(t-butoxycarbonyl)amidino]-4-piperidinyl]propionyl]-3-piperidinyl]carbonyl]-β-alaninbenzylester und 176 mg Pd/C werden in 14,1 ml Ameisensäure 18 Stunden unter Wasserstoff gerührt. Der Katalysator wird abfiltriert und mit Ameisensäure-Wasser 1:1 gewaschen. Das Filtrat wird eingedampft, der Rückstand in Wasser gelöst und die Lösung wieder eingedampft. Der Rückstand wird mit Aethanol-Methanol an Kieselgel chromatographiert. Man erhält 254 mg rac-N-[[1-[3-(1-Amidino-4-piperidinyl)propionyl]-3-piperidinyl]carbonyl]-β-alanin, MS: 382 (100, M+H).

Der Ausgangsester kann wie folgt hergestellt werden:

a) 4-Piperidinpropionsäure wird in t-Butanol und 2N Natronlauge mit N,N'-Bis(t-butoxycarbonyl)-S-methylisothioharnstoff zu 3-[1-[(E/Z)-N,N'-Bis(t-butoxycarbonyl)amidino]-4-piperidinyl]propionsäure umgesetzt.

b) Diese wird mit rac-N-(3-Piperidinylcarbonyl)-β-alaninbenzylester zum Ausgangsester gekoppelt, MS: 672 (12, M+H).

Beispiel 16

115 mg N-[[(S)-1-[p-[N-[(Benzyloxy)carbonyl]amidino]benzoyl]-2-pyrrolidinyl]acetyl]-β-alanin und 50 mg Pd/C werden in 2,5 ml Essigsäure 4 Stunden unter Wasserstoff gerührt. Der Katalysator wird abfiltriert und die Lösung eingedampft. Der Rückstand wird in Wasser gelöst, wieder eingedampft und mit Methanol an Kieselgel chromatographiert. Man erhält 46 mg N-[[(S)-1-(p-Amidinobenzoyl)-2-pyrrolidinyl]acetyl]-β-alanin, Smp. >250°C.

Die Ausgangsverbindung kann wie folgt hergestellt werden:

a) (S)-1-[(Benzyloxy)carbonyl]-2-pyrrolidinessigsäure wird mit β-Alanin-t-butylester zu N-[[(S)-1-[(Benzyloxy)carbonyl]-2-pyrrolidinyl]acetyl]-β-alanin-t-butylester gekoppelt. MS: 391 (69, M+H).

b) Durch Hydrierung in Essigsäure erhält man daraus das Acetat des N-[(2-Pyrrolidinyl)acetyl]-β-alanin-t-butylesters.

c) Dieser wird in Methylenchlorid/Wasser in Gegenwart von Natriumbicarbonat mit p-Amidinobenzoylchlorid und anschliessend mit Chlorameisensäurebenzylester zum N-[[(S)-1-[p-[N-[(Benzyloxy)carbonyl]amidino]benzoyl]-2-pyrrolidinyl]acetyl]β-alanin-t-butylester, Smp. 127-128°C, umgesetzt.

d) In Ameisensäure erhält man daraus die Ausgangsverbindung, MS: 481 (100, M+H).

Beispiel 17

300 mg Benzyl-(S)-3-[[N-[p-[N-(t-butoxycarbonyl)amidino]benzoyl]-β-alanyl]amino]-3-[(p-methoxyphenäthyl) carbamoyl]propionat und 75 mg Pd/C werden 4 1/2 Stunden in 6 ml Ameisensäure unter Wasserstoff gerührt. Der Katalysator wird abfiltriert, das Filtrat eingedampft, der Rückstand in Wasser aufgenommen und wieder eingedampft. Die kristalline Substanz wird in Wasser suspendiert, unter Rühren mit Ammoniak auf pH 8 eingestellt und danach abgenutscht. Man erhält 151 mg (S)-3-[[N-(p-Amidinobenzoyl)-β-alanyl]amino-3-[(p-methoxyphenäthyl)carbamoyl] propionsäure als Hydrat (1:1), Smp. 217°C.

Der Ausgangsester kann wie folgt erhalten werden:

a) β-Alaninbenzylester wird in Methylenchlorid/Wasser/ Natriumbicarbonat mit p-Amidinobenzoylchlorid und anschliessend mit Di-t-butyldicarbonat und Natriumcarbonat zu N-[p-[N-(t-Butoxycarbonyl)amidino]benzoyl]-β-alaninbenzylester, Smp. 127-128°C, umgesetzt.

b) Durch katalytische Hydrierung erhält man daraus das N-[p-[N-(t-Butoxycarbonyl)amidino]benzoyl]-β-alanin, MS: 336 (21, M+H).

c) N-(t-Butoxycarbonyl)asparaginsäure-4-benzylester wird mit 2-(4-Methoxyphenyl)äthylamin zu t-Butyl-[(S)-2-[(benzyloxy)carbonyl]-1-[(p-methoxyphenäthyl)carbamoyl]äthyl]carbamat, Smp. 103-104°C, gekoppelt.

d) In Trifluoressigsäure erhält man daraus das Trifluoracetat des 3-[(p-Methoxyphenäthyl)carbamoyl]-β-alaninbenzylesters.

e) Letzterer wird mit dem bei b) beschriebenen Produkt zum Ausgangsester, Smp. 150°C (Zers.), gekoppelt.

Beispiel 18

Analog Beispiel 8 erhält man aus N-[3-[(Benzyloxycarbonyl]-N-[N-[p-[N-[(benzyloxy)carbonyl]amidino]benzoyl]-β-alanyl]-L-alanyl]-L-leucinisopropylester den N-[N-[N-(p-Amidinobenzoyl)-β-alanyl]-L-α-aspartyl]-L-leucinisopropylester als Hydrat (1:1,3), Smp. 234°C (Zers.).

Der Ausgangsester kann auf folgendem Weg erhalten werden:

a) N-(t-Butoxycarbonyl)-L-leucin wird in Pyridin mit Dicyclohexylcarbodiimid, p-Toluolsulfonsäure und Isopropanol in den N-(t-Butoxycarbonyl)-L-leucinisopropylester übergeführt, $[\alpha]_D$ = -33° (MeOH, c = 0,5).

b) In Trifluoressigsäure erhält man daraus das Trifluoracetat des L-Leucinisopropylesters.

c) Dieses wird mit N-(t-Butoxycarbonyl)-L-asparaginsäure-4-benzylester zum N-[3-[(Benzyloxy)carbonyl]-N-(t-butoxycarbonyl)-L-alanyl]-L-leucinisopropylester gekoppelt, MS: 479 (25, M+H).

d) Nach Spaltung der t-Butoxycarbonylschutzgruppe und Kopplung mit N-(t-Butoxycarbonyl)-β-alanin erhält man daraus den N-[3-[(Benzyloxy)carbonyl]-N-[N-(t-butoxycarbonyl)-β-alanyl]-L-alanyl]-L-leucinisopropylester, Smp. 103-104°C.

e) Dieser wird in Trifluoressigsäure von der t-Butoxycarbonylschutzgruppe befreit und danach in Methylenchlorid/Wasser/Natriumbicarbonat mit p-Amidinobenzoylchlorid und anschliessend mit Chlorameisensäurebenzylester in den Ausgangsester übergeführt, Smp. 176-177°C.

Beispiel 19

Analog Beispiel 8 erhält man aus N-[[(R)-1-[p-[N-[(Benzyloxy)carbonyl]amidino]benzoyl]-3-pyrrolidinyl]carbonyl]-β-alaninbenzylester das N-[[(R)-1-(p-Amidinobenzoyl)-3-pyrrolidinyl]carbonyl]-β-alanin als Hydrat (4:3), Smp. >250°C, $[\alpha]_D$ = -4,13° (1N HCl, c = = 0,46%).

Der Ausgangsester kann folgendermassen hergestellt werden:

a) (R)-1-[(R)-α-Methylbenzyl]-3-pyrrolidinmethanol, Di-t-butyldicarbonat und Pd/C werden 20 Stunden in Aethanol unter Wasserstoff gerührt. Man erhält (R)-3-(Hydroxymethyl)l-pyrrolidincarbonsäure-t-butylester, Smp. 35°C, $[\alpha]_D$ = +19,5° (Methanol, c = 1,0).

b) Daraus erhält man mit Pyridiniumdichromat in DMF die (R)-1-(t-Butoxycarbonyl)-3-pyrrolidincarbonsäure, Smp. 135-138°C, $[\alpha]_D$ = -15° (MeOH, c = 1,0).

c) Diese wird mit β-Alaninbenzylester zum N-[[(R)-1-(t-Butoxycarbonyl)-3-pyrrolidinyl]carbonyl]-β-alaninbenzylester gekoppelt, Smp. 83-84°C, $[\alpha]_D$ = -3,4° (MeOH, c = 1,0).

d) In Trifluoressigsäure erhält man daraus das Trifluoracetat des N-[[(R)-3-pyrrolidinyl]carbonyl]-β-alaninbenzylesters.

e) Dieser wird in Methylenchlorid/Wasser/Natriumbicarbonat mit p-Amidinobenzoylchlorid und anschliessend mit Chlorameisensäurebenzylester zum Ausgangsester umgesetzt, $[\alpha]_D$ = +2,2° (MeOH, c = 0,5).

Beispiel 20

Analog Beispiel 16 erhält man aus Benzyl-(S)-3-[[D/L-N-[p-[N-[(benzyloxy)carbonyl]amidino]benzoyl]-2-methyl-β-alanyl]amino]succinamat das N-[N-(p-Amidinobenzoyl)-2-methyl-β-alanyl]-L-asparaginsäure-α-amid (Epimere 2:1), Smp. 280°C.

Der Ausgangsester kann wie folgt hergestellt werden:

a) Benzyl-(S)-3-(1-t-butoxyformamido)succinamat wird mit Trifluoressigsäure entschützt und dann mit DL-N-(t-Butoxycarbonyl)-2-methyl-β-alanin gekoppelt. Man erhält t-Butyl[(RS)-2-[[(S)-2-[(benzyloxy)carbonyl]-1-carbamoyläthyl]carbamoyl]propyl]carbamat, Smp. 135-136°C.

b) Nach Spaltung der t-Butoxycarbonylschutzgruppe in Trifluoressigsäure koppelt man in Methylenchlorid/Wasser/Natriumbicarbonat mit p-Amidinobenzoylchlorid und setzt schliesslich mit Chlorameisensäurebenzylester zum Ausgangsester (Epimere 2:1) um, Smp. 178,5-179,5°C.

Beispiel 21

548 mg N-[N-[p-[N-(t-Butoxycarbonyl)amidino]phenyl]acetyl]-β-alanyl]-β-alaninbenzylester werden in 11 ml Ameisensäure 18 Stunden stehen gelassen. Nach Zugabe von 137 mg Pd/C wird 4 Stunden unter Wasserstoff gerührt. Der Katalysator wird abfiltriert und das Filtrat eingedampft. Der Rückstand wird in Wasser gelöst und die Lösung wieder eingedampft. Der Rückstand wird in Wasser suspendiert und mit Ammoniak auf pH 8 eingestellt, dann abgenutscht und getrocknet. Man erhält 290 mg N-[N-[(p-Amidinophenyl)acetyl]-β-alanyl]-β-alanin als Hydrat (1:1), Smp. 286°C (Zers.).

Das Ausgangsmaterial. Smp. 262°C (Zers.), erhält man durch Umsetzen von N-(β-Alanyl)-β-alaninbenzylester in DMF/Triäthylamin mit p-Amidinophenylacetylchlorid und anschliessende Reaktion mit Di-t-butyldicarbonat.

Beispiel 22

535 mg rac-N-[[1-[p-[N-(t-Butoxycarbonyl)amidino]phenylacetyl]-3-piperidinyl]carbonyl]-β-alaninbenzylester werden 19 Stunden in 11 ml Ameisensäure stehen gelassen. Das Lösungsmittel wird abgedampft, der Rückstand mit Wasser abgedampft und aus Acetonitril umkristallisiert. Es werden 340 mg rac-N-[[[1-(p-Amidinophenyl)acetyl]-3-piperidinyl]carbonyl]-β-alaninbenzylester-formiat (1:1), Smp. 97-98°C, erhalten.

Den Ausgangsester, MS: 551 (9, M+H), erhält man durch Kopplung von rac-N-[(3-Piperidinyl)carbonyl]-β-alaninbenzylester mit p-Amidinophenylacetylchlorid in DMF/Triäthylamin und anschliessende Reaktion mit Di-t-butyldicarbonat.

Beispiel 23

535 mg rac-N-[[1-[p-[N-(t-Butoxycarbonyl)amidino]phenylacetyl]-3-piperidinyl ]carbonyl]-β-alaninbenzylester werden in 11 ml Ameisensäure 19 Stunden stehen gelassen, mit 134 mg Pd/C versetzt und 4 Stunden unter Wasserstoff gerührt. Die Lösung wird filtriert und eingedampft, der Rückstand in Wasser gelöst und die Lösung wieder eingedampft. Das Produkt wird in Acetonitril verrührt, abgenutscht und getrocknet. Es werden 272 mg rac-N-[[1-[(p-Amidinophenyl) acetyl]-3-piperidinyl]carbonyl]-β-alanin erhalten, MS: 361 (41. M+H).

Beispiel 24

1127 mg N-[3-[(Benzyloxy)carbonyl]-N-[N-[3-[1-[(E oder Z)-N,N'-bis(t-butoxycarbonyl)amidino]-4-piperidinyl]propionyl]-β-alanyl]-L-alanyl]-3-phenyl-L-alaninbenzylester werden 21 Stunden in 22,5 ml Ameisensäure stehen gelassen. Nach Zugabe von 282 mg Pd/C wird 5 Stunden unter Wasserstoff gerührt. Die Lösung wird filtriert und eingedampft, der Rückstand in Wasser gelöst und die Lösung wieder eingedampft. Der Rückstand wird in Wasser verrührt, abgenutscht und getrocknet. Es werden 543 mg N-[N-[N-[3-(1-Amidino-4-piperidinyl)propionyl]-β-alanyl]-L-α-aspartyl] 3-phenyl-L-alanin, Smp. 246°C (Zers.), erhalten.

Der Ausgangsester kann wie folgt hergestellt werden:

a) N-(t-Butoxycarbonyl)-β-alanin wird mit N-[3-[(Benzyloxy)carbonyl]-L-alanyl]-3-phenyl-L-alaninbenzylester zum N-[3-[(Benzyloxy)carbonyl]-N-[N-(t-butoxycarbonyl)-β-alanyl]-L-alanyl]-3-phenyl-L-alaninbenzylester, Smp. 124-125°C, gekoppelt.

b) Nach Spaltung der t-Butoxycarbonylschutzgruppe koppelt man mit 3-[1-[N,N'-Bis (t-butoxycarbonyl)amidino]-4-piperidinyl]propionsäure zum Ausgangsester, Essigester-Solvat 1:1, Smp. 100°C (Zers.).

Beispiel 25

1,3 g 3-[(Benzyloxy)carbonyl]-N-[5-[p-[N-[(benzyloxy)carbonyl]amidino]benzamido]valeryl]-L-alaninisobutylester und 325 mg Pd/C werden in 25 ml Essigsäure 5 1/2 Stunden unter Wasserstoff gerührt. Die Lösung wird filtriert und

eingedampft und der Rückstand nacheinander mit Wasser, Methanol und Aethanol abgedampft. Das Produkt wird in Acetonitril mit Ammoniak auf pH 8 eingestellt, verrührt und abgenutscht. Es werden 596 mg N-[5-(p-Amidinobenzamido)-valeryl]-L-α-asparaginsäureisobutylester als Hydrat (1:1) erhalten, Smp. 162-166°C.

Der Ausgangsester. Smp. 127,5-129,5°C, kann wie folgt hergestellt werden:

a) N-(t-Butoxycarbonyl)-L-asparaginsäure-4-benzylester wird in Pyridin mit Dicyclohexylcarbodiimid, p-Toluolsulfonsäure und Isobutylalkohol in den N-(t-Butoxycarbonyl)-3-[(benzyloxy)carbonyl]-L-alaninisobutylester überführt, MS: 323 (8, M-$C_4H_8$).

b) Nach Spaltung der t-Butoxycarbonylgruppe in Trifluoressigsäure koppelt man mit 5-(1-t-Butoxyformamido)valeriansäure zum 3-[(Benzyloxy)carbonyl]-N-[5-(1-t-butoxyformamido)valeryl]-L-alaninisobutylester, Smp. 64-66°C.

c) Dieser wird mit Trifluoressigsäure entschützt und dann in Methylenchlorid/Wasser/Natriumbicarbonat mit p-Amidinobenzoylchlorid und anschliessend mit Chlorameisensäurebenzylester zum Ausgangsester umgesetzt.

Beispiel 26

562mg(S)-β-[[DL-N-[p-[N-[(Benzyloxy)carbonyl]amidino]benzoyl]-3-methyl-β-alanyl]amino]-γ-oxo-1-pyrrolidinbuttersäurebenzylester und 140 mg Pd/C werden in 11 ml Essigsäure 2 Stunden unter Wasserstoff gerührt. Die filtrierte Lösung wird eingedampft und der Rückstand nacheinander mit Wasser, Methanol und Aethanol abgedampft. Schliesslich wird in Aethanol mit Ammoniak auf pH 8 eingestellt, verrührt und abgenutscht. Es werden 289 mg (S)-β-[[DL-N-(p-Amidinobenzoyl)-3-methyl-β-alanyl]amino]-γ-oxo-1-pyrrolidinbuttersäure als Hydrat (2:3), Smp. 222-224°C, erhalten.

Der Ausgangsester kann auf folgendem Weg hergestellt werden:

a) (S)-β-(1-t-Butoxyformamido)-γ-oxo-1-pyrrolidinbuttersäurebenzylester wird mit Trifluoressigsäure entschützt und mit (RS)-3-(1-t-Butoxyformamido)buttersäure zum (S)-β-[(RS)-3-(1-t-Butoxyformamido)butyramido]-γ-oxo-1-pyrrolidinbuttersäurebenzylester, Smp. 104-105°C, gekoppelt.

b) Daraus erhält man nach Spaltung der t-Butoxycarbonylgruppe in Trifluoressigsäure und Umsetzen mit p-Amidinobenzoylchlorid und anschliessend mit Chlorameisensäurebenzylester in Methylenchlorid/Wasser/Natriumbicarbonat den Ausgangsester, MS: 642 (100, M+H).

Beispiel 27

Analog Beispiel 8 erhält man aus dem DL-N-[N-[p-[N-[(Benzyloxy)carbonyl]amidino]benzoyl]-β-alanyl]-3-methyl-β-alaninbenzylester das DL-N-[N-(p-Amidinobenzoyl)-β-alanyl]-3-methyl-β-alanin als Hydrat (3:1), Smp. 291°C (Zers.).

Der Ausgangsester, Smp. 179-180°C, kann wie folgt hergestellt werden:

a) N-(t-Butoxycarbonyl)-β-alanin wird mit DL-3-Aminobuttersäurebenzylester zum DL-N-[N-(t-Butoxycarbonyl)-β-alanyl]-3-methyl-β-alaninbenzylester, Smp. 70-72°C, gekoppelt.

b) Daraus erhält man mit Trifluoressigsäure den DL-N-(β-Alanyl)-3-methyl-β-alaninbenzylester. c) Dieser wird in Methylenchlorid/Wasser/Natriumbicarbonat mit p-Amidinobenzoylchlorid und anschliessend mit Chlorameisensäurebenzylester zum Ausgangsester umgesetzt.

Beispiel 28

Analog Beispiel 8 erhält man aus N-[3-[(Benzyloxy)carbonyl]-N-[N-[p-[N-[(benzyloxy)carbonyl]amidino]benzoyl]-β-alanyl]-L-serinäthylester den N-[N-[N-(p-Amidinobenzoyl)-β-alanyl]-L-α-aspartyl]-L-serinäthylester als Hydrat (2:7), Smp. 201-203°C.

Der Ausgangsester, Smp. 177-179°C, kann wie folgt hergestellt werden:

a) N-(t-Butoxycarbonyl)-L-asparaginsäure-4-benzylester wird mit L-Serinäthylester zum N-[3-[(Benzyloxy)carbonyl]-N-(t-butoxycarbonyl)-L-alanyl]-L-serinäthylester, Smp. 96-97°C, gekoppelt.

b) Nach Spaltung der t-Butoxycarbonylgruppe koppelt man mit N-(t-Butoxycarbonyl)-β-alanin zum N-[3-[(Benzyloxy)carbonyl]-N-[N-(t-butoxycarbonyl)-β-alanyl]-L-alanyl]-L-serinäthylester, Smp. 132-134°C.

c) Dieser wird in Trifluoressigsäure entschützt und anschliessend in Methylenchlorid/Wasser/Natriumbicarbonat mit p-Amidinobenzoylchlorid und schliesslich mit Chlorameisensäurebenzylester zum Ausgangsester umgesetzt.

Beispiel 29

Analog Beispiel 8 erhält man aus N-[N-[p-[DL-N-[(Benzyloxy)carbonyl]amidino]benzoyl]-2-phenyl-β-alanyl]-β-alanin-benzylester das N-[DL-N-(p-Amidinobenzoyl)-2-phenyl-β-alanyl]-β-alanin als Hydrat (4:1), Smp. 276°C.

Der Ausgangsester, Smp. 173-174°C, wird wie folgt hergestellt:

a) DL-2-Phenyl-β-alanin wird in t-Butanol und Natronlauge mit Di-t-butyldicarbonat zum DL-N-(t-Butoxycarbonyl)-2-phenyl-β-alanin umgesetzt, Smp. 147-148°C.

b) Dieses wird mit β-Alaninbenzylester zu N-[DL-N-(t-Butoxycarbonyl)-2-phenyl-β-alanyl]-β-alaninbenzylester, Smp. 115-116°C, gekoppelt.

c) Nach Spaltung der Schutzgruppe wird in Methylenchlorid/ Wasser/Natriumbicarbonat mit p-Amidinobenzoylchlorid und anschliessend mit Chlorameisensäurebenzylester zum Ausgangsester umgesetzt.

Beispiel 30

Analog Beispiel 8 erhält man aus N-[N-[p-[N-[(Benzyloxy)carbonyl]amidino]benzoyl]-β-alanyl]-DL-2-methyl-β-alaninbenzylester das DL-N-[N-(p-Amidinobenzoyl)-β-alanyl]-2-methyl-β-alanin als Hydrat (3:1), Smp. >300°C.

Der Ausgangsester, Smp. 159-160°C, wird wie folgt erhalten:

a) N-(t-Butoxycarbonyl)-β-alanin wird mit [DL-2-Methyl-β-alaninbenzylester zum DL-N-[N-(t-Butoxycarbonyl)-β-alanyl]-2-methyl-β-alaninbenzylester gekoppelt, MS: 365 (47, M+H).

b) Nach Spaltung der Schutzgruppe wird in Methylenchlorid/ Wasser/Natriumbicarbonat mit p-Amidinobenzoylchlorid und anschliessend mit Chlorameisensäurebenzylester zum Ausgangsester umgesetzt.

Beispiel 31

Analog Beispiel 11 erhält man aus dem N-[N-[[(R)-1-[p-[N-[(Benzyloxy)carbonyl]amidino]benzoyl]-3-pyrrolidinyl] carbonyl]-3-[(benzyloxy)carbonyl]-L-alanyl]-3-phenyl-L-alaninbenzylester nach Abdampfen mit Wasser und Verrühren in Aethanol das N-[N-[[(R)-1-(p-Amidinobenzoyl)-3-pyrrolidinyl]carbonyl]-L-α-aspartyl]-3-phenyl-L-alanin-acetat (2:1) als Hydrat (3:2), Smp. 215°C.

Den Ausgangsester erhält man wie folgt:

a) (R)-1-(t-Butoxycarbonyl)-3-pyrrolidincarbonsäure wird mit N-[3-[(Benzyloxy)carbonyl]-L-alanyl]-3-phenyl-L-alaninbenzylester zum N-[N-[[(R)-1-(t-Butoxycarbonyl)-3-pyrrolidinyl]carbonyl]-3-[(benzyloxy)carbonyl]-L-alanyl]-3-phenyl-L-alaninbenzylester, Smp. 84-85°C, gekoppelt.

b) Nach Entfernung der t-Butoxycarbonylgruppe in Trifluoressigsäure setzt man in Methylenchlorid/Wasser/Natriumbicarbonat mit p-Amidinobenzoylchlorid und anschliessend mit Chlorameisensäurebenzylester zum Ausgangsester, Smp. 144-145°C, um.

Beispiel 32

Analog Beispiel 7 erhält man aus N-[N-[p-[N-[(Benzyloxy)carbonyl]amidino]benzoyl]-β-alanyl]-DL-2-phenyl-β-alaninbenzylester das DL-N-[N-(p-Amidinobenzoyl)-β-alanyl]-2-phenyl-β-alanin als Hydrat (1:1), Smp. 243-245°C.

Der Ausgangsester kann wie folgt erhalten werden:

a) N-(t-Butoxycarbonyl)-2-phenyl-β-alanin wird in Aceton mit Benzylbromid und Kaliumcarbonat 17 Stunden am Rückfluss gekocht. Man erhält DL-N-(t-Butoxycarbonyl)-2-phenyl-β-alaninbenzylester, Smp. 58-59°C.

b) Nach Spaltung der t-Butoxycarbonylgruppe koppelt man mit N-(t-Butoxycarbonyl)-β-alanin zum DL-N-[N-(t-Butoxycarbonyl)-β-alanyl]-2-phenyl-β-alaninbenzylester, Smp. 84,5-86°.C.

c) Dieser wird in Trifluoressigsäure ins Trifluoracetat des -(β-Alanyl)-2-phenyl-β-alaninbenzylesters übergeführt.

d) Letzterer wird in Methylenchlorid/Wasser/Natriumbicarbonat mit p-Amidinobenzoylchlorid und anschliessend mit Chlorameisensäurebenzylester zum Ausgangsester, Smp. 165-166°C, umgesetzt.

Beispiel 33

Analog Beispiel 11 erhält man aus dem p-[2-[[3-[(Benzyloxy)carbonyl]-N-[N-[p-[N-[(benzyloxy)carbonyl]amidino] benzoyl]-β-alanyl]-L-alanyl]amino]äthyl]benzoesäurebenzylester die p-[2-[[N-[N-(p-Amidinobenzoyl)-β-alanyl]-L-α-aspartyl]amino]äthyl]benzoesäure als Hydrat (3:7), Smp. 194-196°C (Methanol/Wasser).

Der Ausgangsester, Smp. 162-163°C, kann wie folgt hergestellt werden:

a) p-(2-Chloräthyl)benzoylchlorid wird in Methylenchlorid mit Benzylalkohol und Pyridin in den p-(2-Chloräthyl) benzoesäurebenzylester übergeführt, MS: 274 (8, M).

b) Mit Natriumazid in DMSO erhält man daraus den p-(2-Azidoäthyl)benzoesäurebenzylester, MS: 281 (2, M).

c) Diesen setzt man in Pyridin mit Triphenylphosphin und anschliessend mit konz. Ammoniak zum p-(2-Aminoäthyl) benzoesäurebenzylester um, MS: 226 (16, M-$CH_2$NH).

d) Durch Kopplung mit N-(t-Butoxycarbonyl)-L-asparaginsäure-4-benzylester erhält man daraus den p-[2-[[3-[(Benzyloxy)carbonyl]-N-(t-butoxycarbonyl)-L-alanyl]amino]äthyl]benzoesäurebenzylester, Smp. 99-100°C.

e) Dieser wird in Trifluoressigsäure entschützt und mit N-(t-Butoxycarbonyl)-β-alanin zum p-[2-[[3-[(Benzyloxy) carbonyl]-N-[N-(t-butoxycarbonyl)-β-alanyl]-L-alanyl]amino]äthyl]benzoesäurebenzylester, Smp. 138-139°C, gekoppelt.

f) Nach Spaltung der t-Butoxycarbonylgruppe in Trifluoressigsäure setzt man in Methylenchlorid/Wasser/Natriumbicarbonat mit p-Amidinobenzoylchlorid und anschliessend mit Chlorameisensäurebenzylester zum Ausgangsester um.

Beispiel 34

Analog Beispiel 7 erhält man aus dem DL-N-[N-[p-[N-[(Benzyloxy)carbonyl]amidino]benzoyl]-β-alanyl]-3-phenyl-β-alaninbenzylester das [DL-N-[N-(p-Amidinobenzoyl)-β-alanyl-3-phenyl-β-alanin als Hydrat (3:1), Smp. 220°C (Zers.).

Den Ausgangsester, Smp. 208°C, erhält man wie folgt:

a) N-(t-Butoxycarbonyl)-β-alanin wird mit DL-3-Phenyl-β-alaninbenzylester zum DL-N-[N-(t-Butoxycarbonyl)-β-alanyl]-3-phenyl-β-alaninbenzylester, Smp. 124,5-126°C, gekoppelt.

b) In Trifluoressigsäure erhält man daraus das Trifluoracetat des DL-N-(β-Alanyl)-3-phenyl-β-alaninbenzylesters.

c) Dieses setzt man in Methylenchlorid/Wasser/Natriumbicarbonat mit p-Amidinobenzoylchlorid und anschliessend mit Chlorameisensäurebenzylester zum Ausgangsester um.

Beispiel 35

Analog Beispiel 11 erhält man aus dem Benzyl-(S)-3-[[N-[p-[N-[(benzyloxy)carbonyl]amidino]benzoyl]-β-alanyl] amino]-N-[p-[[(benzyloxy)carbonyl]methoxy]phenäthyl]succinamat die [p-[2-[[N-[N-(p-Amidinobenzoyl)-β-alanyl]-L-α-aspartyl]amino]äthyl]phenoxy]essigsäure als Hydrat (2:7), Smp. 210-213°C.

Der Ausgangsester, Smp. 172-174°C, kann wie folgt hergestellt werden:

a) t-Butyl-[2-(4-hydroxyphenyl)äthyl]carbamat, Bromessigsäurebenzylester und Kaliumcarbonat werden in Aceton erhitzt. Man erhält t-Butyl-[p-[[(benzyloxy)carbonyl]methoxy]phenäthyl]carbamat, MS: 385 (0,5, M).

b) Nach Spaltung der t-Butoxycarbonylschutzgruppe koppelt man mit N-(t-Butoxycarbonyl)asparaginsäure-4-benzylester zum Benzyl-(S)-N-[p-[[(benzyloxy)carbonyl]methoxy]phenäthyl]-3-[1-t-butoxyformamido]succinamat, Smp. 178,5-180,5°C.

c) Dieses wird in Trifluoressigsäure entschützt und mit N-(t-Butoxycarbonyl)-β-alanin zum t-Butyl-[2-[[(S)-2-[(benzyloxy)carbonyl]-1-[[p-[[(benzyloxy)carbonyl]methoxy]phenäthyl]carbamoyl]äthyl]carbamoyl]äthyl]carbamat, Smp. 123-124°C, gekoppelt.

d) Nach Spaltung der t-Butoxycarbonylschutzgruppe wird in Methylenchlorid/Wasser/Natriumbicarbonat mit p-Amidinobenzoylchlorid und anschliessend mit Chlorameisensäurebenzylester zum Ausgangsester umgesetzt.

Beispiel 36

a) Eine Lösung von 280 mg 1-[N-(p-Cyanobenzoyl)-β-alanyl]-4-piperidinessigsäure und 1 ml Triäthylamin in 15 ml Pyridin wird mit Schwefelwasserstoff gesättigt. Nach 36 Stunden wird die Lösung eingedampft und der Rückstand in Essigester/Wasser suspendiert. Filtration und Trocknen des unlöslichen Materials ergibt 255 mg 1-[N-[p-(Thiocarbamoyl)benzoyl]-β-alanyl]-4-piperidinessigsäure.

b) Eine Lösung von 150 mg der Vorstufe in 15 ml Aceton wird mit 1 ml Methyljodid 3 Stunden auf Siedetemperatur erhitzt. Nach Abkühlen der Lösung auf Raumtemperatur fallen 130 mg 1-[N-[p-[1-(Methylthio)formimidoyl]benzoyl]-β-alanyl]-4-piperidinessigsäure hydrojodid (1:1), Smp. 206-207°C, aus

c) 100 mg 1-[N-[p-[1-(Methylthio)formimidoyl]benzoyl]-β-alanyl]-4-piperidinessigsäure und 30 mg Ammoniumacetat werden in 10 ml Methanol 3 Stunden bei Siedetemperatur gehalten. Nach Abkühlen auf Raumtemperatur wird die Lösung filtriert, eingedampft und mit Diäthyläther versetzt. Das ausgefallene Oel wird nach Abdekantieren des Lösungsmittels mit Wasser/Methanol (10:1) an Kieselgel RP 18 chromatographiert. Man erhält 24 mg 1-[N-(p-Amidinobenzoyl)-β-alanyl]-4-piperidinessigsäure-hydrojodid (10:1). Smp. 206°C.

Das Ausgangsnitril kann wie folgt erhalten werden:

a) 4,96 g 4-Cyanobenzoylchlorid und 2,67 g β-Alanin werden in 450 ml Natriumbicarbonatlösung (2%) 4 Stunden bei Raumtemperatur gerührt und mit konzentrierter Schwefelsäure angesäuert (pH 6). Die Lösung wird eingedampft und mit Essigsäureäthylester extrahiert. Trocknen und Eindampfen der organischen Phase ergibt einen Rückstand, der mit Diisopropyläther zu 4,69 g N-(p-Cyanobenzoyl)-β-alanin, Smp. 155-157°C führt.

b) Die Kopplung von 635 mg N-(p-Cyanobenzoyl)-β-alanin mit 540 mg 4-Piperidinessigsäure ergibt nach Chromatographie an Kieselgel RP 18 mit THF/Wasser (85:15) 300 mg 1-[N-(p-Cyanobenzoyl)-β-alanyl]-4-piperidinessigsäure, MS: 344 $(M+H)^+$.

Beispiel 37

400 mg t-Butyl-4[N-(p-Amidinobenzoyl)-β-alanyl]-1-piperazinacetat werden in 15 ml Methylenchlorid und 15 ml Trifluoressigsäure gerührt. Die Lösung wird eingedampft, der Rückstand in 5 ml Aethanol suspendiert und das ungelöste Material abfiltriert. Das Filtrat wird mit Essigester versetzt und der Niederschlag abgenutscht und getrocknet. Chromatographie des Rohprodukts an Kieselgel RP 18 mit Wasser führt zu 38 mg 4-[N-(p-Amidinobenzoyl)-β-alanyl]-1-piperazinessigsäure-trifluoracetat (5:8), Smp. 157-158°C.

Das Ausgangsmaterial kann wie folgt hergestellt werden:

a) 2,23 g N-Benzyloxycarbonyl-β-alanin werden mit 2,58 g Piperazin gekoppelt. Der Eindampfrückstand wird in THF suspendiert, das ungelöste Material abfiltriert und das Filtrat eingedampft. Chromatographie des Rückstands an Kieselgel RP 18 mit Wasser/Methanol (2-5%) ergibt 2,51 g Benzyl-[2-(4--piperazinylcarbonyl)äthyl]carbamat, MS: 291 $(M^{+\bullet})$.

b) 600 mg der Vorstufe, 0,3 ml Bromessigsäure-t-butylester und 25 mg Tetrabutylammoniumhydrogensulfat werden in 10 ml Toluol gelöst und während 1 Stunde mit 10 ml 50%-iger Natronlauge gerührt. Die organische Phase wird mit Wasser gewaschen und eingedampft. Chromatographie des Rückstands an Kieselgel mit Essigester/Methanol (9:1) ergibt 480 mg t-Butyl-4-[N-[(benzyloxy)carbonyl]-β-alanyl]-1-piperazinacetat, MS: 406 $(M+H)^+$.

c) Die Vorstufe wird in Gegenwart von 200 mg Pd/C in Aethanol 1 Stunde hydriert. Der Katalysator wird abfiltriert

und das Filtrat eingedampft. Man erhält 290 mg t-Butyl-4-β-alanyl-1-piperazinacetat, MS: 271 ($M^+$).

d) Die Vorstufe und 341 mg p-Amidinobenzoylchlorid werden in 20 ml Methylenchlorid und 10 ml gesättigter Natriumbicarbonatlösung gerührt. Die organische Phase wird abgetrennt, eingedampft und der Rückstand in Essigester suspendiert. Abnutschen und Trocknen der Kristalle ergibt 400 mg t-Butyl-4-[N-(p-amidinobenzoyl)-β-alanyl]-1-piperazinacetat, MS: 418 $(M+H)^+$.

Beispiel 38

Analog Beispiel 37 werden 1,6 g N-[N-[N-(p-Amidinobenzoyl)-β-alanyl]-3-(t-butoxycarbonyl)-L-alanyl]-L-valin-t-butylester entschützt. Das Rohprodukt wird an Kieselgel RP 18 mit Wasser/THF (95:5) chromatographiert. Man erhält 867 mg N-[N-[N-(p-Amidinobenzoyl)-β-alanyl]-L-α--aspartyl]-L-valin-trifluoracetat, Smp. 162-163°C.

Der Ausgangsester kann wie folgt hergestellt werden:

a) N-Benzyloxycarbonyl-L-asparaginsäure-4-t-butylester und L-Valin-t-butylester hydrochlorid werden zu N-[N-[(Benzyloxy)carbonyl]-3-(t-butoxycarbonyl)-L-alanyl]-L-valin-t-butylester gekoppelt, Smp. 75°C (d).

b) Dieser wird analog zu Beispiel 37c entschützt. Man erhält N-[3-t-butoxycarbonyl)-L-alanyl]-L-valin-t-butylester, Smp. 71°C.

c) 2,8 g der Vorstufe werden mit 1,78 g N-[(Benzyloxy)carbonyl]-β-alanin gekoppelt und das Rohprodukt mit Essigester an Kieselgel chromatographiert. Man erhält 2,24 g N-[N-[N-[(Benzyloxy)carbonyl]-β-alanyl]-3-(t-butoxycarbonyl)-L-alanyl]-L-valin-t-butylester, Smp. 126°C.

d) Entschützen von 2,2 g der Vorstufe analog zu Beispiel 37c) führt zu 1,61 g N-[N-β-Alanyl-3-(t-butoxycarbonyl)-L-alanyl]-L-valin-t-butylester.

e) Reaktion von 1 g p-Amidinobenzoylchlorid mit 1,61 g der Vorstufe nach der Vorschrift 37d ergibt 1,62 g Ausgangsester.

Beispiel 39

Analog Beispiel 37 werden 1,4 g N-[N-[N-(p-Amidinobenzoyl)-β-alanyl]-3-(t-butoxycarbonyl)-L-alanyl]-3-(p-t--butoxyphenyl)-L-alanin-t-butylester entschützt. Das Produkt wird in Aethanol suspendiert, nicht lösliches Material abfiltriert und das Filtrat mit Aether versetzt. Abnutschen und Waschen des Niederschlags mit 20 ml Isopropanol/Aethanol (1:1) ergibt 801 mg N-[N-[N-(p-Amidinobenzoyl)-β-alanyl]-L-α-aspartyl]-3-(p-hydroxyphenyl)-L-alanin-trifluoracetat (2:5), Smp. 202-204°C (d).

Der Ausgangsester kann wie folgt hergestellt werden:

a) N-[(9H-Fluoren-9-yloxy)carbonyl]-3-(t-butoxycarbonyl) -L-alanin und 3-(p-t-Butoxyphenyl)-L-alanin-t-butylester werden zu N-[N-[(9H-Fluoren-9-yloxy)carbonyl]-3-(t-butoxycarbonyl)-L-alanyl]-3-(p-t-butoxyphenyl)-L-alanin-t-butylester gekoppelt.

b) 3,2 g der Vorstufe werden in 50 ml DMF und 5 ml Piperidin gerührt und die Reaktionslösung wird eingedampft. Der Rückstand wird in Methanol suspendiert und unlösliches Material abfiltriert. Das Filtrat wird eingedampft und mit Essigester an Kieselgel chromatographiert. Man erhält 2 g 3-(t-Butoxyphenyl)-N-[3-(p-t-butoxycarbonyl)-L-alanyl]-L-alanin-t-butylester.

c) Die Vorstufe wird mit 0,96 g N-Benzyloxycarbonyl-β-alanin gekoppelt und das Rohprodukt mit Essigester an Kieselgel chromatographiert. Man erhält 2,23 g N-[N-[N-[(Benzyloxy)carbonyl]-β-alanyl]-3-(t-butoxycarbonyl)-L-alanyl]-3-(p-t-butoxyphenyl)-L-alanin-t-butylester, MS: 670 $(M+H)^+$.

d) Entschützen von 2,1 g der Vorstufe wie im Beispiel 37c) ergibt 1,67 g N-[N-[N-β-Alanyl]-3-(t-butoxycarbonyl)-L-alanyl] -3-(p-t-butoxyphenyl)-L-alanin-t-butylester, MS: 536 $(M+H)^+$.

e) Die Kopplung von 1,5 g der Vorstufe mit 0,6 g p-Amidinobenzoylchlorid wie in Beispiel 37d) ergibt 1,6 g Ausgangsester, MS: 682 $(M+H)^+$.

Beispiel 40

Analog Beispiel 24 wird das N-[N-[N-[(5-Amidino -2-pyridyl)carbonyl]-β-alanyl]-L-α-aspartyl]-3-phenyl-L-alanin, Smp. 222-223°C, hergestellt.

Der entsprechende Ausgangsester kann folgendermassen hergestellt werden:

a) N-[3-[(Benzyloxy)carbonyl]-N-[N-(t-butoxycarbonyl)-β-alanyl]-L-alanyl]-3-phenyl-L-alaninbenzylester wird in Methylenchlorid/Trifluoressigsäure entschützt und mit 5-Cyan-2-pyridincarbonsäure zum N-[3-[(Benzyloxy)carbonyl] -N-[N-[(5-cyano-2-pyridyl)carbonyl]-β-alanyl]-L-alanyl] -3-phenyl-L-alaninbenzylester, Smp. 157-158°C, gekoppelt.

b) Daraus erhält man mit Schwefelwasserstoff und Triäthylamin in Pyridin den N-[3-[(Benzyloxy)carbonyl] -N-[N-[[5-(thiocarbamoyl)-2-pyridyl]carbonyl]-β-alanyl] -L-alanyl]-3-phenyl-L-alaninbenzylester, Smp. 131-132°C.

c) Dieser wird zunächst mit Methyljodid in Aceton, dann mit Ammoniumacetat und Essigsäure in Methanol und schliesslich mit Di-t-butyl-dicarbonat und Triäthylamin in DMF zum N-[3-[(Benzyloxy)carbonyl]-N-[N-[[5-[N-t-butoxycarbonyl)amidino]-2-pyridyl]carbonyl]-β-alanyl]-L-alanyl]-3-phenyl -L-alaninbenzylester umgesetzt, MS: 779 (11, M+H).

Beispiel A

Eine Verbindung der Formel I kann man in an sich bekannter Weise als Wirkstoff zur Herstellung von Tabletten folgender Zusammensetzung verwenden:

| | pro Tablette |
|---|---|
| Wirkstoff | 200 mg |
| mikrokristalline Cellulose | 155 mg |
| Maisstärke | 25 mg |
| Talk | 25 mg |
| Hydroxypropylmethylcellulose | 20 mg |
| | 425 mg |

Beispiel B

Eine Verbindung der Formel I kann man in an sich bekannter Weise als Wirkstoff zur Herstellung von Kapseln folgender Zusammensetzung verwenden:

| | pro Kapsel |
|---|---|
| Wirkstoff | 100,0 mg |
| Maisstärke | 20,0 mg |
| Milchzucker | 95,0 mg |
| Talk | 4,5 mg |
| Magnesiumstearat | 0.5 mg |
| | 220,0 mg |

**Patentansprüche**

**Patentansprüche für folgende Vertragsstaaten : AT, BE, CH, DE, DK, FR, GB, IT, LI, LU, NL, SE**

**1.** Peptidbindungen enthaltende Essigsäurederivate der Formel

$$H_2N(NH)C\text{-}X\text{-}Y\text{-}CO\text{-}Z\text{-}CH(Q^1)COOQ^2 \qquad (I)$$

worin

- $Q^1$ Wasserstoff, Methyl oder Phenyl,
- $Q^2$ Wasserstoff. Phenyl-nieder-alkyl oder unter physiologischen Bedingungen spaltbares nieder-Alkyl,
- X 1,4-Phenylen, 2,5- oder 3,6-Pyridylen oder über das C-Atom in 4-Stellung an die Gruppe Y gebundenes 1,4-Piperidinylen,
- Y eine Gruppe der Formel

$$-(CH_2)_{0\text{-}2}\text{-}CONHCH(Q^3)(CH_2)_{1\text{-}3}\text{-} \quad (Y^1)$$

$$-CONHCH_2CH(Q^4)\text{-} \quad (Y^2)$$

$$-(CH_2)_2NHCOCH_2\text{-} \quad (Y^3)$$

$$-NHCO(CH_2)_3\text{-} \quad (Y^4)$$

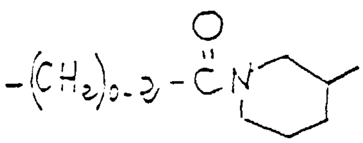

$(Y^5)$

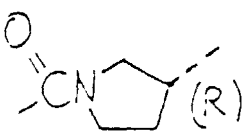

$(Y^6)$

oder

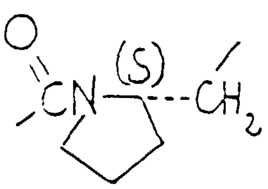

$(Y^7)$

- $Q^3$ Wasserstoff, Methyl, Phenyl, -COOH oder $-CONH(CH_2)_2\text{-}COOH$,
- $Q^4$ Wasserstoff, Methyl oder Phenyl,
- Z eine 1,4-Piperazinylengruppe, eine über das N-Atom in 1-Stellung an die CO-Gruppe gebundene 1,4-Piperidinylengruppe oder eine Gruppe der Formel

$$-NHCH(R^1)\text{-}$$

oder

$$-NHCH(COR^2)-$$

$R^1$ Wasserstoff, Methyl, Phenyl oder -COO-nieder-Alkyl,
$R^2$ der Rest einer über die Aminogruppe gebundenen α-Aminocarbonsäure oder eines Esters davon, oder eine Gruppe der Formel $-NHCH_2CH_2-Ar$, oder
$-CO-R^2$ eine Carbamoylgruppe oder eine Pyrrolidinoyl- oder Piperidinoylgruppe,
Ar Phenyl oder durch nieder-Alkyl, nieder-Alkoxy, -COOH, -COO-nieder-Alkyl, $-O(CH_2)_{1-4}-COOH$ oder $O(CH_2)_{1-4}$ -COO-nieder-Alkyl, substituiertes Phenyl ist,

sowie Hydrate oder Solvate und physiologisch verwendbare Salze davon.

**2.** Verbindungen nach Anspruch 1 und der Formel

$$H_2N(HN)C-X-Y^a-CONHCH(R^{11})CH_2COOQ^{21} \qquad \text{I-A}$$

worin

X 1,4-Phenylen oder über das C-Atom in 4-Stellung an die Gruppe $Y^a$ gebundenes 1,4-Piperidinylen,
$Y^a$ eine Gruppe der Formel

$$-(CH_2)_{0-1}-CONHCH(Q^a)\ (CH_2)_{1-2}- \qquad (Y^{11})$$

$$-(CH_2)_2NHCOCH_2- \qquad (Y^3)$$

$$-NHCO(CH_2)_3- \qquad (Y^4)$$

oder

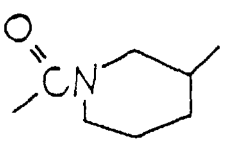

$(Y^{51})$

$Q^a$ Wasserstoff oder Phenyl,
$R^{11}$ Wasserstoff oder $-CO-R^{22}$,
$R^{22}$ der Rest einer über die Aminogruppe gebundenen α-Aminocarbonsäure oder eines Esters davon,
$Q^{21}$ Wasserstoff oder unter physiologischen Bedingungen spaltbares nieder-Alkyl ist,

sowie Hydrate oder Solvate und physiologisch verwendbare Salze davon.

**3.** Verbindungen nach Anspruch 1, worin X. Z, $Q^1$ und $Q^2$ die in Anspruch 1 angegebene Bedeutung haben und Y eine Gruppe der Formel $Y^1$ ist, insbesondere eine solche der Formel

$$-CONH(CH_2)_{2-4}-,$$

$$-CH_2CONH(CH_2)_2-,$$

$-CONHCH(C_6H_5)CH_2-$,

$-CONHCH(CONHCH_2CH_2COOH)CH_2-$,

$-CONHCH(COOH)CH_2-$,

oder

$-CONHCH(CH_3)CH_2-$.

**4.** Verbindungen nach Anspruch 1, worin X, Y, $Q^1$ und $Q^2$ die in Anspruch 1 angegebene Bedeutung haben und Z eine Gruppe der Formel $-NHCH_2-$, $-NHCH(CH_3)-$, $-NHCH(C_6H_5)-$, -NHCH(COO-Isobutyl)-, -NHCH(CO-Val)-, -NHCH(CO-Phe)-, -NHCH(CO-Tyr)-, $-NHCH(CO-Ser-OC_2H_5)-$, -NHCH(CO-Leu-O-Isopropyl)-, $-NHCH(CONHCH_2CH_2-C_6H_4-OCH_3)-$, $-NHCH(CONHCH_2CH_2-C_6H_4-COOH)-$, $-NHCH(CONHCH_2CH_2-C_6H_4-OCH_2COOH)-$, $-NHCH(CONH_2)-$ oder -NHCH(Pyrrolidinoyl)- ist.

**5.** Verbindungen nach Anspruch 2 aus der Gruppe der folgenden:

N-[N-[4-(p-Amidinobenzamido)butyryl]-L-α-aspartyl]-L-valin,
N-[N-(p-Amidinobenzoyl)-β-alanyl]-β-alanin und insbesondere
N-[N-[N-(p-Amidinobenzoyl)-β-alanyl]-L-α-aspartyl]-3-phenyl-L-alanin.

**6.** Verbindungen nach Anspruch 2 aus der Gruppe der folgenden:

N-[N-[N-(1-Amidino-4-piperidinylcarbonyl)-β-alanyl]-L-α-aspartyl]-3-phenyl-L-alanin
N-[N-[N-(p-Amidinophenylacetyl)-β-alanyl]-L-α-aspartyl]-3-phenyl-L-alanin
N-[N-[4-(p-Amidinophenylcarbamoyl)butyryl]-L-α-aspartyl]-3-phenyl-L-alanin
N-[N-[(p-Amidinophenylcarbamoyl)acetyl]-L-α-aspartyl]-3-phenyl-L-alanin
rac-N-[1-(p-Amidinobenzoyl)-3-piperidinylcarbonyl]-β-alanin
N-[4-(p-Amidinobenzamido)butyryl]-β-alanin und
N-[(DL)-N-(p-Amidinobenzoyl)-3-phenyl-β-alanyl]-β-alanin.

**7.** Verbindungen nach Anspruch 1 aus der Gruppe der folgenden:

N-[N-[N-(p-Amidinobenzoyl)-β-alanyl]-L-α-aspartyl]-L-leucinisopropylester,
N-[N-[N-(p-Amidinobenzoyl)-β-alanyl]-L-α-aspartyl]-L-valin,
N-[N-[N-(p-Amidinobenzoyl)-β-alanyl]-L-α-aspartyl]-3-(p-hydroxyphenyl)-L-alanin,
N-[N-[5-(p-Amidinobenzamido)valeryl]-L-α-aspartyl]-3-phenyl-L-alanin,
N-[5-(p-Amidinobenzamido)valeryl]-L-α-asparaginsäure-isobutylester,
N-[N-[N-(p-Amidinobenzoyl)-β-alanyl]-L-α-aspartyl]-L-serinäthylester und
N-[N-[[(R)-1-(p-Amidinobenzoyl)-3-pyrrolidinyl]carbonyl]-L-α-aspartyl]-3-phenyl-L-alanin.

**8.** Verbindungen nach Anspruch 1 aus der Gruppe der folgenen:

N,N'-[[(S)-(p-Amidinobenzamido)äthylen]dicarbonyl]di-β-alanin,
N2-(p-Amidinobenzoyl)-N4-(2-carboxyäthyl)-L-asparagin, N-[5-(p-Amidinobenzamido)valeryl]-β-alanin,
rac-N-[[1-[3-(1-Amidino-4-piperidinyl)propionyl]-3-piperidinyl]carbonyl]-β-alanin,
N-[[(S)-1-(p-Amidinobenzoyl)-2-pyrrolidinyl]acetyl]-β-alanin,
(S)-3-[[N-(p-Amidinobenzoyl)-β-alanyl]amino-3-[(p-methoxyphenäthyl)carbamoyl]propionsäure,
N-[[(R)-1-(p-Amidinobenzoyl)-3-pyrrolidinyl]carbonyl]-β-alanin,
N-[N-(p-Amidinobenzoyl)-2-methyl-β-alanyl]-L-asparaginsäure-α-amid,
N-[N-[(p-Amidinophenyl)acetyl]-β-alanyl]-β-alanin,
rac-N-[[[1-(p-Amidinophenyl)acetyl]-3-piperidinyl]carbonyl]-β-alaninbenzylester,
rac-N-[[1-[(p-Amidinophenyl)acetyl]-3-piperidinyl)carbonyl]-β-alanin,

N-[N-[N-[3-(1-Amidino-4-piperidinyl)propionyl]-β-alanyl] L-α-aspartyl]-3-phenyl-L-alanin,
(S)-β-[[DL-N-(p-Amidinobenzoyl)-3-methyl-β-alanyl]amino]-γ-oxo-1-pyrrolidinbuttersäure,
DL-N-[N-(p-Amidinobenzoyl)-β-alanyl]-3-methyl-β-alanin,
N-[DL-N-(p-Amidinobenzoyl)-2-phenyl-β-alanyl]-β-alanin,
DL-N-[N-(p-Amidinobenzoyl)-β-alanyl]-2-methyl-β-alanin,
DL-N-[N-(p-Amidinobenzoyl)-β-alanyl]-2-phenyl-β-alanin,
p-[2-[[N-[N-(p-Amidinobenzoyl)-β-alanyl]-L-α-aspartyl]amino]äthyl]benzoesäure,
DL-N-[N-(p-Amidinobenzoyl)-β-alanyl-3-phenyl-β-alanin,
[p-[2-[[N-[N-(p-Amidinobenzoyl)-β-alanyl]-L-α-aspartyl]amino]äthyl]phenoxy]essigsäure,
1-[N-(p-Amidinobenzoyl)-β-alanyl]-4-piperidinessigsäure,
4-[N-(p-Amidinobenzoyl)-β-alanyl]-1-piperazinessigsäure und N-[N-[N-[(5-Amidino-2-pyridyl)carbonyl]-β-alanyl] -L-α-aspartyl]-3-phenyl-L-alanin.

**9.** Essigsäurederivate der Formel

$$X'\text{-}Y\text{-}CO\text{-}Z\text{-}CH(Q^1)\text{-}COOQ^2 \qquad II'$$

worin X' in 4-Stellung durch eine gegebenenfalls geschützte Amidinogruppe substituiertes Phenyl oder 4-Piperidinyl ist, und Y, Z, $Q^1$ und $Q^2$ die gleiche Bedeutung wie in Anspruch 1 haben, wobei das Molekül mindestens eine leicht spaltbare Estergruppe oder geschützte Amidinogruppe enthält.

**10.** Verbindungen nach einem der Ansprüche 1-8 zur Verwendung als pharmazeutische Wirkstoffe.

**11.** Verfahren zur Herstellung der Verbindungen nach Anspruch 1, dadurch gekennzeichnet, dass man

a) aus einer Verbindung der Formel

$$X'\text{-}Y\text{-}CO\text{-}Z'\text{-}CH(Q^1)\text{-}COO\text{-}Q^5 \qquad II$$

worin

| | |
|---|---|
| $Q^1$ und Y | die im Anspruch 1 angegebene Bedeutung hat, |
| X' | in 4-Stellung durch eine gegebenenfalls gechützte Amidinogruppe substituiertes Phenyl oder 4-Piperidinyl ist, und |
| Z' bzw. $Q^5$ | die gleiche Bedeutung wie Z bzw. $Q^2$ im Anspruch 1 haben, |

zumindest eine Schutzgruppe abspaltet, oder

b) in einem Nitril der Formel

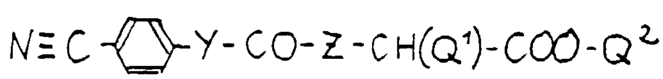

(III)

die Nitrilgruppe in die Amidinogruppe überführt, und gewünschtenfalls eine Verbindung der Formel I in ein physiologisch verträgliches Salz überführt oder ein Salz einer Verbindung der Formel I in die freie Säure oder Base überführt.

**12.** Pharmazeutische Präparate, insbesondere für die Behandlung oder Prophylaxe von Krankheiten, die durch die Bindung von adhäsiven Proteinen an Blutplättchen, sowie durch die Blutplättchenaggregation und die Zell-Zell-Adhäsion verursacht sind, enthaltend eine Verbindung nach Anspruch 1 oder 2 als Wirkstoff.

**13.** Verwendung einer Verbindung nach Anspruch 1 oder 2 zur Herstellung von Arzneimitteln zur Behandlung oder Prophylaxe von Krankheiten, die durch die Bindung von adhäsiven Proteinen an Blutplättchen, sowie durch die

Blutplättchenaggregation und die Zell-Zell-Adhäsion verursacht sind, insbesondere zur Behandlung oder Prophylaxe von Blutplättchenthromben, Thrombose, Hirnschlag, Herzinfarkt, Entzündung oder Arteriosklerose, oder als Antitumor-Mittel oder als Mittel für die Wundheilung.

**Patentansprüche für folgende Vertragsstaaten : ES, GR**

1. Verfahren zur Herstellung der Peptidbindungen enthaltenden Essigsäurederivate der Formel

$$H_2N(NH)C\text{-}X\text{-}Y\text{-}CO\text{-}Z\text{-}CH(Q^1)COOQ^2 \qquad (I)$$

worin

$Q^1$ Wasserstoff, Methyl oder Phenyl,
$Q^2$ Wasserstoff, Phenyl-nieder-alkyl oder unter physiologischen Bedingungen spaltbares nieder-Alkyl,
X 1,4-Phenylen, 2,5- oder 3,6-Pyridylen oder über das C-Atom in 4-Stellung an die Gruppe Y gebundenes 1,4-Piperidinylen,
Y eine Gruppe der Formel

$$-(CH_2)_{0-2}\text{-}CONHCH(Q^3)(CH_2)_{1-3}\text{-} \qquad (Y^1)$$

$$-CONHCH_2CH(Q^4)\text{-} \qquad (Y^2)$$

$$-(CH_2)_2NHCOCH_2\text{-} \qquad (Y^3)$$

$$-NHCO(CH_2)_3\text{-} \qquad (Y^4)$$

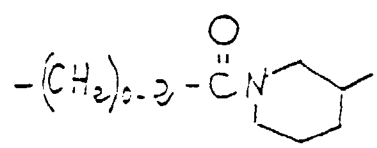

$(Y^5)$

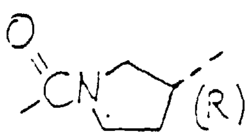

$(Y^6)$

oder

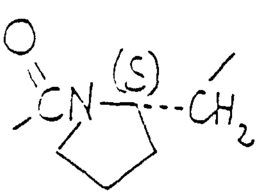

$(Y^7)$

$Q^3$ Wasserstoff, Methyl, Phenyl, -COOH oder $-CONH(CH_2)_2\text{-}COOH$

$Q^4$ Wasserstoff, Methyl oder Phenyl,
Z eine 1,4-Piperazinylengruppe, eine über das N-Atom in 1-Stellung an die CO-Gruppe gebundene 1,4-Piperidinylengruppe oder eine Gruppe der Formel

$$-NHCH(R^1)-$$

oder

$$-NHCH(COR^2)-$$

$R^1$ Wasserstoff, Methyl, Phenyl oder -COO-nieder-Alkyl,
$R^2$ der Rest einer über die Aminogruppe gebundenen $\alpha$-Aminocarbonsäure oder eines Esters davon, oder eine Gruppe der Formel
$NHCH_2CH_2$-Ar, oder
-CO-$R^2$ eine Carbamoylgruppe oder eine Pyrrolidinoyl- oder Piperidinoylgruppe,
Ar Phenyl oder durch nieder-Alkyl, nieder-Alkoxy, -COOH, -COO-nieder-Alkyl, $O(CH_2)_{1-4}$-COOH oder $O(CH_2)_{1-4}$ -COO-nieder-Alkyl, substituiertes Phenyl ist.

sowie Hydrate oder Solvate und physiologisch verwendbare Salze davon, dadurch gekennzeichnet, dass man

a) aus einer Verbindung der Formel

$$X'\text{-}Y\text{-}CO\text{-}Z'\text{-}CH(Q^1)\text{-}COO\text{-}Q^5 \qquad II$$

worin

| | |
|---|---|
| $Q^1$ und Y | die vorstehend angegebene Bedeutung haben, |
| X' | in 4-Stellung durch eine gegebenenfalls geschützte Amidinogruppe substituiertes Phenyl oder 4-Piperidinyl ist, und |
| Z' bzw. $Q^5$ | die gleiche Bedeutung wie Z bzw. $Q^2$ vorstehend haben, zumindest eine Schutzgruppe abspaltet, oder |

b) in einem Nitril der Formel

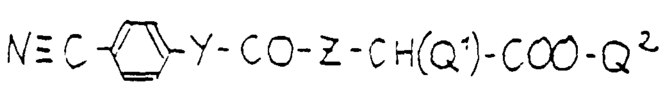

(III)

die Nitrilgruppe in die Amidinogruppe überführt, und gewünschtenfalls eine Verbindung der Formel I in ein physiologisch verträgliches Salz überführt oder ein Salz einer Verbindung der Formel I in die freie Säure oder Base überführt.

**2.** Verfahren nach Anspruch 1, dadurch gekennzeichnet, dass man Verbindungen der Formel

$$H_2N(HN)C\text{-}X\text{-}Y^a\text{-}CONHCH(R^{11})CH_2COOQ^{21} \qquad I\text{-}A$$

worin

X 1,4-Phenylen oder über das C-Atom in 4-Stellung an die Gruppe $Y^a$ gebundenes 1,4-Piperidinylen,
$Y^a$ eine Gruppe der Formel

$-(CH_2)_{0-1}-CONHCH(Q^a)(CH_2)_{1-2}-$ ($Y^{11}$)

$-(CH_2)_2NHCOCH_2-$ ($Y^3$)

$-NHCO(CH_2)_3-$ ($Y^4$)

oder

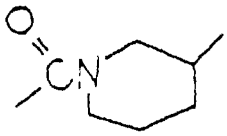

($Y^{51}$)

$Q^a$ Wasserstoff oder Phenyl,
$R^{11}$ Wasserstoff oder -CO-$R^{22}$,
$R^{22}$ der Rest einer über die Aminogruppe gebundenen α-Aminocarbonsäure oder eines Esters davon,
$Q^{21}$ Wasserstoff oder unter physiologischen Bedingungen spaltbares nieder-Alkyl ist,

sowie Hydrate oder Solvate und physiologisch verwendbare Salze davon, herstellt.

**3.** Verfahren nach Anspruch 1, worin X, Z, $Q^1$ und $Q^2$ die in Anspruch 1 angegebene Bedeutung haben und Y eine Gruppe der Formel $Y^1$ ist, insbesondere eine solche der Formel

$-CONH(CH_2)_{2-4}-$,

$-CH_2CONH(CH_2)_2-$,

$-CONHCH(C_6H_5)CH_2-$,

$-CONHCH(CONHCH_2CH_2COOH)CH_2$ -,

$-CONHCH(COOH)CH_2-$

oder

$-CONHCH(CH_3)CH_2-$.

**4.** Verfahren nach Anspruch 1. worin X, Y, $Q^1$ und $Q^2$ die in Anspruch 1 angegebene Bedeutung haben und Z eine Gruppe der Formel $-NHCH_2-$, $NHCH(CH_3)-$, $-NHCH(C_6H_5)-$, -NHCH(COO-Isobutyl)-, -NHCH(CO-Val)-, -NHCH (CO-Phe)-, -NHCH(CO-Tyr)-, -NHCH(CO-Ser-$OC_2H_5$)-, -NHCH(CO-Leu-O-Isopropyl)-, -NHCH($CONHCH_2CH_2$-$C_6H_4$-$OCH_3$)-, -NHCH($CONHCH_2CH_2$-$C_6H_4$-COOH)-. -NHCH($CONHCH_2CH_2$-$C_6H_4$-$OCH_2COOH$)-, -NHCH ($CONH_2$)- oder -NHCH(Pyrrolidinoyl)- ist.

**5.** Verfahren nach Anspruch 2, dadurch gekennzeichnet, dass man Verbindungen aus der Gruppe der folgenden herstellt:

N-[N-[4-(p-Amidinobenzamido)butyryl]-L-α-aspartyl]-L-valin,
N-[N-(p-Amidinobenzoyl)-β-alanyl]-β-alanin und insbesondere

N-[N-[N-(p-Amidinobenzoyl)-β-alanyl]-L-α-aspartyl]-3-phenyl-L-alanin.

**6.** Verfahren nach Anspruch 2, dadurch gekennzeichnet, dass man Verbindungen aus der Gruppe der folgenden herstellt:

N-[N-[N-(1-Amidino-4-piperidinylcarbonyl)-β-alanyl]-L-α-aspartyl]-3-phenyl-L-alanin
N-[N-[N-(p-Amidinophenylacetyl)-β-alanyl]-L-α-aspartyl]-3-phenyl-L-alanin
N-[N-[4-(p-Amidinophenylcarbamoyl)butyryl]-L-α-aspartyl]-3-phenyl-L-alanin
N-[N-[(p-Amidinophenylcarbamoyl)acetyl]-L-α-aspartyl]-3-phenyl-L-alanin
rac-N-[1-(p-Amidinobenzoyl)-3-piperidinylcarbonyl]-β-alanin
N-[4-(p-Amidinobenzamido)butyryl]-β-alanin und
N-[(DL)-N-(p-Amidinobenzoyl)-3-phenyl-β-alanyl]-β-alanin.

**7.** Verfahren nach Anspruch 1, dadurch gekennzeichnet, dass man Verbindungen aus der Gruppe der folgenden herstellt:

N-[N-[N-(p-Amidinobenzoyl)-β-alanyl]-L-α-aspartyl]-L-leucinisopropylester,
N-[N-[N-(p-Amidinobenzoyl)-β-alanyl]-L-α-aspartyl]-L-valin,
N-[N-[N-(p-Amidinobenzoyl)-β-alanyl]-L-α-aspartyl]-3-(p-hydroxyphenyl)-L-alanin,
N-[N-[5-(p-Amidinobenzamido)valeryl]-L-α-aspartyl]-3-phenyl-L-alanin,
N-[5-(p-Amidinobenzamido)valeryl]-L-α-asparaginsäure-isobutylester,
N-[N-[N-(p-Amidinobenzoyl)-β-alanyl]-L-α-aspartyl]-L-serinäthylester und
N-[N-[[(R)-1-(p-Amidinobenzoyl)-3-pyrrolidinyl]carbonyl]-L-α-aspartyl]-3-phenyl-L-alanin.

**8.** Verfahren nach Anspruch 1, dadurch gekennzeichnet, dass man Verbindungen aus der Gruppe der folgenden herstellt:

N,N'-[[(S)-(p-Amidinobenzamido)äthylen]dicarbonyl]di-β-alanin,
N2-(p-Amidinobenzoyl)-N4-(2-carboxyäthyl)-L-asparagin,
N-[5-(p-Amidinobenzamido)valeryl]-β-alanin,
rac-N-[[1-[3-(1-Amidino-4-piperidinyl)propionyl]-3-piperidinyl]carbonyl]-β-alanin,
N-[[(S)-1-(p-Amidinobenzoyl)-2-pyrrolidinyl]acetyl]-β-alanin,
(S)-3-[[N-(p-Amidinobenzoyl)-β-alanyl]amino-3-[(p-methoxyphenäthyl)carbamoyl]propionsäure,
N-[[(R)-1-(p-Amidinobenzoyl)-3-pyrrolidinyl]carbonyl]-β-alanin,
N-[N-(p-Amidinobenzoyl)-2-methyl-β-alanyl]-L-asparaginsäure-α-amid,
N-[N-[(p-Amidinophenyl)acetyl]-β-alanyl]-β-alanin,
rac-N-[[[1-(p-Amidinophenyl)acetyl]-3-piperidinyl]carbonyl]-β-alaninbenzylester,
rac-N-[[1-[(p-Amidinophenyl)acetyl]-3-piperidinyl)carbonyl]-β-alanin,
N-[N-[N-[3-(1-Amidino-4-piperidinyl)propionyl]-β-alanyl]-L-α-aspartyl]-3-phenyl-L-alanin,
(S)-β-[[DL-N-(p-Amidinobenzoyl)-3-methyl-β-alanyl]amino]-γ-oxo-1-pyrrolidinbuttersäure,
DL-N-[N-(p-Amidinobenzoyl)-β-alanyl]-3-methyl-β-alanin,
N-[DL-N-(p-Amidinobenzoyl)-2-phenyl-β-alanyl]-β-alanin,
DL-N-[N-(p-Amidinobenzoyl)-β-alanyl]-2-methyl-β-alanin,
DL-N-[N-(p-Amidinobenzoyl)-β-alanyl]-2-phenyl-β-alanin,
p-[2-[[N-[N-(p-Amidinobenzoyl)-β-alanyl]-L-α-aspartyl]amino]äthyl]benzoesäure,
DL-N-[N-(p-Amidinobenzoyl)-β-alanyl-3-phenyl-β-alanin,
[p-[2-[[N-[N-(p-Amidinobenzoyl)-β-alanyl]-L-α-aspartyl]amino]äthyl]phenoxy]essigsäure.
1-[N-(p-Amidinobenzoyl)-β-alanyl]-4-piperidinessigsäure,
4-[N-(p-Amidinobenzoyl)-β-alanyl]-1-piperazinessigsäure und N-[N-[N-[(5-Amidino-2-pyridyl)carbonyl]-β-alanyl] -L-α-aspartyl]-3-phenyl-L-alanin.

**9.** Verfahren zur Herstellung von pharmazeutischen Präparaten, insbesondere für die Behandlung oder Prophylaxe von Krankheiten, die durch die Bindung von adhäsiven Proteinen an Blutplättchen, sowie durch die Blutplättchenaggregation und die Zell-Zell-Adhäsion verursacht sind, dadurch gekennzeichnet, dass man eine Verbindung nach Anspruch 1 oder 2 in eine galenische Darreichungsform bringt.

**10.** Verwendung einer Verbindung nach Anspruch 1 oder 2 zur Herstellung von Arzneimitteln zur Behandlung oder Prophylaxe von Krankheiten, die durch die Bindung von adhäsiven Proteinen an Blutplättchen, sowie durch die

Blutplättchenaggregation und die Zell-Zell-Adhäsion verursacht sind, insbesondere zur Behandlung oder Prophylaxe von Blutplättchenthromben, Thrombose, Hirnschlag, Herzinfarkt, Entzündung oder Arteriosklerose, oder als Antitumor-Mittel oder als Mittel für die Wundheilung.

**11.** Essigsäurederivate der Formel

$$X'\text{-}Y\text{-}CO\text{-}Z\text{-}CH(Q^1)\text{-}COOQ^2 \qquad II'$$

worin X' in 4-Stellung durch eine gegebenenfalls geschützte Amidinogruppe substituiertes Phenyl oder 4-Piperidinyl ist, und Y, Z, $Q^1$ und $Q^2$ die gleiche Bedeutung wie in Anspruch 1 haben, wobei das Molekül mindestens eine leicht spaltbare Estergruppe oder geschützte Amidinogruppe enthält.

## Claims

**Claims for the following Contracting States : AT, BE, CH, DE, DK, FR, GB, IT, LI, LU, NL, SE**

**1.** Acetic acid derivatives containing peptide bonds of the formula

$$H_2N(NH)C\text{-}X\text{-}Y\text{-}CO\text{-}Z\text{-}CH(Q^1)COOQ^2 \qquad (I)$$

in which

- $Q^1$ Is hydrogen, methyl or phenyl,
- $Q^2$ is hydrogen, phenyl-lower alkyl or lower alkyl which can be cleaved under physiological conditions,
- X is 1,4-phenylene, 2,5- or 3,6-pyridylene or 1,4-piperidinylene bonded via the C atom in the 4-position to the group Y,
- Y is a group of the formula

$$-(CH_2)_{0\text{-}2}\text{-}CONHCH(Q^3)(CH_2)_{1\text{-}3}\text{-} \qquad (Y^1)$$

$$-CONHCH_2CH(Q^4)\text{-} \qquad (Y^2)$$

$$-(CH_2)_2NHCOCH_2\text{-} \qquad (Y^3)$$

$$-NHCO(CH_2)_3\text{-} \qquad (Y^4)$$

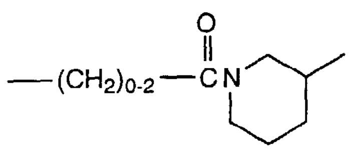

($Y^5$)

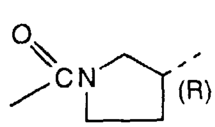

($Y^6$)

or

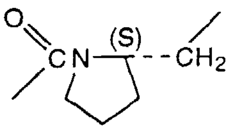

$(Y^7)$

| | |
|---|---|
| $Q^3$ | is hydrogen, methyl, phenyl, -COOH or $-CONH(CH_2)_2-COOH$, |
| Q4 | is hydrogen, methyl or phenyl, |
| Z | is a 1,4-piperazinylene group, a 1,4-piperidinylene group bonded via the N-atom in the 1-position to the CO group, or a group of the formula |

$$-NHCH(R^1)-$$

or

$$-NHCH(COR^2)-$$

| | |
|---|---|
| $R^1$ | is hydrogen, methyl, phenyl or -COO-lower alkyl, |
| $R^2$ | is the residue of an α-aminocarboxylic acid bonded via the amino group or of an ester thereof, or a group of the formula $-NHCH_2CH_2-Ar$, or |
| $-CO-R^2$ | is a carbamoyl group or a pyrrolidinoyl or piperidinoyl group, |
| Ar | is phenyl or phenyl substituted by lower alkyl, lower alkoxy, -COOH, -COO-lower alkyl, $-O(CH_2)_{1-4}-COOH$ or $-O(CH_2)_{1-4}-COO-$ lower alkyl |

as well as hydrates or solvates and physiologically utilizable salts thereof.

**2.** Compounds according to claim 1 and of the formula

$$H_2N(HN)C-X-Y^a-CONHCH(R^{11})CH_2COOQ^{21} \quad \text{I-A}$$

in which

| | |
|---|---|
| X | is 1,4-phenylene or 1,4-piperidinylene bonded via the C atom in the 4-position to the group $Y^a$, |
| $Y^a$ | is a group of the formula |

$$-(CH_2)_{0-1}-CONHCH(Q^a)(CH_2)_{1-2}- \quad (Y^{11})$$

$$-(CH_2)_2-NHCOCH_2- \quad (Y^3)$$

$$-NHCO(CH_2)_3- \quad (Y^4)$$

or

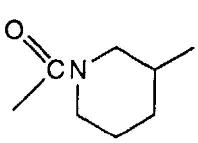

$(Y^{51})$

$Q^a$ is hydrogen or phenyl,
$R^{11}$ is hydrogen or -CO-$R^{22}$,
$R^{22}$ is the residue of an α-aminocarboxylic acid bonded via the amino group or of an ester thereof,
$Q^{21}$ is hydrogen or lower alkyl which can be cleaved under physiological conditions,

as well as hydrates or solvates and physiologically utilizable salts thereof.

**3.** Compounds according to claim 1, in which X, Z, $Q^1$ and $Q^2$ have the meaning indicated in claim 1 and Y is a group of formula $Y^1$, in particular one of the formula

$$-CONH(CH_2)_{2\text{-}4}-,$$

$$-CH_2CONH(CH_2)_2-$$

$$-CONHCH(C_6H_5)CH_2-,$$

$$-CONHCH(CONHCH_2CH_2COOH)CH_2-,$$

$$-CONHCH(COOH)CH_2-$$

or

$$-CONHCH(CH_3)CH_2-.$$

**4.** Compounds according to claim 1, in which X, Y, $Q^1$ and $Q^2$ have the meaning indicated in claim 1 and Z is a group of the formula $-NHCH_2-$, $-NHCH(CH_3)-$, $-NHCH(C_6H_5)-$, -NHCH(COO-isobutyl)-, -NHCH(CO-Val)-, -NHCH(CO-Phe)-, -NHCH(CO-Tyr)-, -NHCH(CO-Ser-$OC_2H_5$)-, -NHCH(CO)-Leu-O-isopropyl)-, -NHCH($CONHCH_2CH_2$-$C_6H_4$-$OCH_3$)-, -NHCH($CONHCH_2CH_2$-$C_6H_4$-COOH)-, -NHCH($CONHCH_2CH_2$-$C_6H_4$-$OCH_2COOH$)-, -NHCH($CONH_2$)- or NHCH(pyrrolidinoyl)-.

**5.** Compounds according to claim 2 from the following group:

N-[N-[4-(p-Amidinobenzamido)butyryl]-L-α-aspartyl]-L-valine,
N-[N-(p-amidinobenzoyl)β-alanyl]-β-alanine and in particular
N-[N-[N-(p-amidinobenzoyl)-β-alanyl]-L-α-aspartyl]-3-phenyl-L-alanine.

**6.** Compounds according to claim 2 from the following group:

N-[N-[N-(1-Amidino-4-piperidinylcarbonyl)-β-alanyl]-L-α-aspartyl]-3-phenyl-L-alanine,
N-[N-[N-(p-amidinophenylacetyl)-β-alanyl]-L-α-aspartyl]-3-phenyl-L-alanine,
N-[N-[4-(p-amidinophenylcarbamoyl)butyryl]-L-α-aspartyl]-3-phenyl-L-alanine,
N-[N-(p-amidinophenylcarbamoyl)acetyl]-L-α aspartyl]-3-phenyl-L-alanine,
rac-N-[p-amidinobenzoyl)-3-piperidinylcarbonyl]-β-alanine,
N-[4-(p-amidinobenzamido)butyryl]-β-alanine and
N-[(DL)-N-(p-amidinobenzoyl)-3-phenyl-β-alanyl]-β-alanine.

**7.** Compounds according to claim 1 from the following group:

N-[N-[N-(p-Amidinobenzoyl)-β-alanyl]-L-α-aspartyl]-L-leucine isopropyl ester,
N-[N-[N-(p-amidinobenzoyl)-β-alanyl]-L-α-aspartyl]-L-valine,
N-[N-[N-(p-amidinobenzoyl)-β-alanyl]-L-α-aspartyl]-3-(p-hydroxyphenyl)-L-alanine,
N-[N-[5-(p-amidinobenzamido)valeryl]-L-α-aspartyl]-3-phenyl-L-alanine,

isobutyl N-[5-(p-amidinobenzamido)valeryl]-L-α-aspartate,
N-[N-[N-(p-amidinobenzoyl)-β-alanyl]-L-α-aspartyl]-L-serine ethyl ester and
N-[N-[[(R)-1-(p-amidinobenzoyl)-3-pyrrolidinyl]carbonyl]-L-α-aspartyl]-3-phenyl-L-alanine.

**8.** Compounds according to claim 1 from the following group:

N,N'-[[(S)-(p-Amidinobenzamido)ethylene]dicarbonyl]di-β-alanine,
N2-(p-amidinobenzoyl)-N4-(2-carboxyethyl)-L-asparagine,
N-[5-(p-amidinobenzamido)valeryl]-β-alanine,
rac-N-[[1-[3-(1-amidino-4-piperidinyl)propionyl]-3-piperidinyl]-carbonyl]-β-alanine,
N-[[(S)-1-(p-amidinobenzoyl)-2-pyrrolidinyl]acetyl]-β-alanine,
(S)-3-[[N-(p-amidinobenzoyl)-β-alanyl] amino-3-[(p-methoxyphenethyl)carbamoyl]propionic acid,
N-[[(R)-1-(p-amidinobenzoyl)-3-pyrrolidinyl] carbonyl]-β-alanine,
N-[N-(p-amidinobenzoyl)-2-methyl-β-alanyl]-L-α-aspartamide,
N-[N-[(p-amidinophenyl)acetyl]-β-alanyl]-β-alanine,
rac-N-[[[1-(p-amidinophenyl)acetyl]-3-piperidinyl)carbonyl]-β-alanine benzyl ester,
rac-N-[[1-[(p-amidinophenyl)acetyl]-3-piperindinyl]-carbonyl]-β-alanine,
N-[N-[N-[3-(1-amidino-4-piperidinyl)propionyl]-β-alanyl]-L-α-aspartyl]-3-phenyl-L-alanine,
(S)-β-[[DL-N-(p-amidinobenzoyl)-3-methyl-β-alanyl]amino]-γ-oxo-1-pyrrolidinebutyric acid,
DL-N-[N-(p-amidinobenzoyl)-β-alanyl]-3-methyl-β-alanine,
N-[DL-N-(p-amidinobenzoyl)-2-phenyl-β-alanyl]-β-alanine,
DL-N-[N-(p-amidinobenzoyl)-β-alanyl]-2-methyl-β-alanine,
DL-N-[N-(p-amidinobenzoyl)-β-alanyl]-2-phenyl-β-alanine,
p-[2-[[N-[N-(p-amidinobenzoyl)-β-alanyl]-L-α-aspartyl]amino]ethyl]-benzoic acid,
DL-N-[N-(p-amidinobenzoyl)-β-alanyl-3-phenyl-β-alanine,
[p-[2-[[N-[N-(p-amidinobenzoyl)-β-alanyl]-L-α-aspartyl] amino]ethyl]phenoxy]acetic acid,
1-[N-(p-amidinobenzoyl)-β-alanyl]-4-piperidineacetic acid,
4-[N-(p-amidinobenzoyl)-β-alanyl]-1-piperazineacetic acid and N-[N-[N-[(5-amidino-2-pyridyl)carbonyl]-β-alanyl]-L-α-aspartyl]-3-phenyl-L-alanine.

**9.** Acetic acid derivatives of the formula

$$X'\text{-}Y\text{-}CO\text{-}Z\text{-}CH(Q^1)\text{-}COOQ^2 \qquad II'$$

in which X' is phenyl or 4-piperidinyl substituted in the 4-position by an optionally protected amidino group and Y, Z, $Q^1$ and $Q^2$ have the same meaning as in claim 1, with the molecule containing at least one easily cleavable ester group or protected amidino group.

**10.** Compounds according to any one of claims 1-8 for use as pharmaceutically active substances.

**11.** A process for the preparation of the compounds according to claim 1, characterized in that

a) at least one protecting group is removed from a compound of the formula

$$X'\text{-}Y\text{-}CO\text{-}Z'\text{-}CH(Q^1)\text{-}COO\text{-}Q^5 \qquad II$$

in which

| | |
|---|---|
| $Q^1$ and Y | have the meaning indicated in claim 1, |
| X' | is phenyl or 4-piperidinyl which is substituted in the 4-position by an optionally protected amidino group and |
| Z' and $Q^5$ | have the same meaning as Z and $Q^2$ in claim 1, or |

b) the nitrile group is converted into the amidino group in a nitrile of the formula

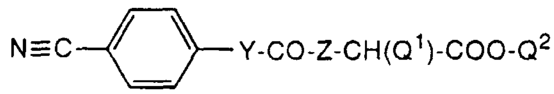 (III)

and, if desired, a compound of the formula I is converted into a physiologically tolerable salt or a salt of formula I is converted into the free acid or base.

**12.** Pharmaceutical preparations, in particular for the treatment or prophylaxis of diseases which are caused by the binding of adhesive proteins to blood platelets, and also by blood platelet aggregation and cell-cell adhesion, containing a compound according to claim 1 or 2 as the active substance

**13.** The use of a compound according to claim 1 or 2 for the production of medicaments for the treatment or prophylaxis of diseases which are caused by the binding of adhesive proteins to blood platelets, and also by blood platelet aggregation and cell-cell adhesion, in particular for the treatment or prophylaxis of blood platelet thrombi, thrombosis, cerebral infarct, myocardial infarct, inflammation or arteriosclerosis, or as antitumour agents or as agents for the healing of wounds.

**Claims for the following Contracting States : ES, GR**

**1.** A process for the preparation of acetic acid derivatives containing peptide bonds of the formula

$$H_2N(NH)C\text{-}X\text{-}Y\text{-}CO\text{-}Z\text{-}CH(Q^1)COOQ^2 \qquad (I)$$

in which

$Q^1$ is hydrogen, methyl or phenyl,
$Q^2$ is hydrogen, phenyl-lower alkyl or lower alkyl which can be cleaved under physiological conditions,
X is 1,4-phenylene, 2,5- or 3,6-pyridylene or 1,4-piperidinylene bonded via the C atom in the 4-position to the group Y,
Y is a group of the formula

$$-(CH_2)_{0\text{-}2}\text{-}CONHCH(Q^3)(CH_2)_{1\text{-}3}\text{-} \qquad (Y^1)$$

$$-CONHCH_2CH(Q^4)\text{-} \qquad (Y^2)$$

$$-(CH_2)NHCOCH_2\text{-} \qquad (Y^3)$$

$$-NHCO(CH_2)_3\text{-} \qquad (Y^4)$$

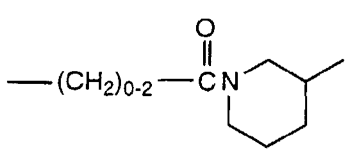 $(Y^5)$

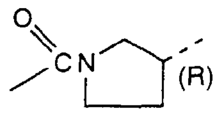 ($Y^6$)

or

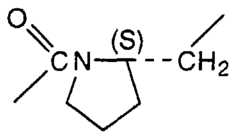 ($Y^7$)

| | |
|---|---|
| $Q^3$ | is hydrogen, methyl, phenyl, -COOH or $CONH(CH_2)_2$-COOH, |
| $Q^4$ | is hydrogen, methyl or phenyl, |
| Z | is a 1,4-piperazinylene group, a 1,4-piperidinylene group bonded via the N atom in the 1-position to the CO group, or a group of the formula |

$$-NHCH(R^1)-$$

or

$$-NHCH(COR^2)-$$

| | |
|---|---|
| $R^1$ | is hydrogen, methyl, phenyl or -COO-lower alkyl, |
| $R^2$ | is the residue of an $\alpha$-aminocarboxylic acid bonded via the amino group or of an ester thereof, or a group of the formula $-NHCH_2CH_2$-Ar, or |
| $-CO-R^2$ | is a carbamoyl group or a pyrrolidinoyl or piperidinoyl group, |
| Ar | is phenyl or phenyl which is substituted by lower alkyl, lower alkoxy, -COOH, -COO-lower alkyl, $-O(CH_2)_{1-4}$-COOH or $-O(CH_2)_{1-4}$-COO-lower alkyl, |

as well as hydrates or solvates and physiologically utilizable salts thereof, characterized in that

a) at least one protecting group is removed from a compound of the formula

$$X'-Y-CO-Z'-CH(Q^1)-COO-Q^5 \qquad II$$

in which

| | |
|---|---|
| $Q^1$ and Y | have the meaning indicated previously, |
| X' | is phenyl or 4-piperidinyl which is substituted in the 4-position by an optionally protected amidino group and |
| Z' resp.$Q^5$ | have the same meaning as Z resp. $Q^2$ previously, |

or

b) the nitrile group is converted into the amidino group in a nitrile of the formula

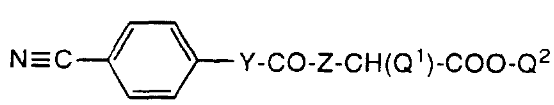 (III)

and, if desired, a compound of formula I is converted into a physiologically tolerable salt or a salt of the compound of the formula I into the free acid or base.

**2.** A process according to claim 1, characterized in that compounds of the formula

$$H_2N(HN)C\text{-}X\text{-}Y^a\text{-}CONHCH(R^{11})CH_2COOQ^{21} \qquad \text{I-A}$$

in which

X is 1,4-phenylene or 1,4-piperidinylene bonded via the C atom in the 4-position to the group $Y^a$,
$Y^a$ is a group of the formula

$$-(CH_2)_{0\text{-}1}\text{-}CONHCH(Q^a)(CH_2)_{1\text{-}2}\text{-} \qquad (Y^{11})$$

$$-(CH_2)_2NHCOCH_2\text{-} \qquad (Y^3)$$

$$-NHCO(CH_2)_3\text{-} \qquad (Y^4)$$

or

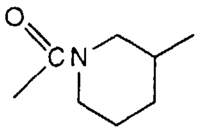

($Y^{51}$)

$Q^a$ is hydrogen or phenyl,
$R^{11}$ is hydrogen or CO-$R^{22}$,
$R^{22}$ is the residue of an α-aminocarboxylic acid bonded via the amino group or an ester thereof,
$Q^{21}$ is hydrogen or lower alkyl which can be cleaved under physiological conditions,

as well as hydrates or solvates and physiologically utilizable salts thereof are prepared.

**3.** A process according to claim 1, in which X, Z, $Q^1$ and $Q^2$ have the meaning indicated in claim 1 and Y is a group of formula $Y^1$, in particular one of the formula

$$-CONH(CH_2)_{2\text{-}4}\text{-},$$

$$-CH_2CONH(CH_2)_2\text{-},$$

$$-CONHCH(C_6H_5)CH_2\text{-}$$

$$-CONHCH(CONHCH_2CH_2COOH)CH_2\text{-},$$

$$-CONHCH(COOH)CH_2\text{-}$$

or

$-CONHCH(CH_3)CH_2-$.

**4.** A process according to claim 1, in which X, Y, $Q^1$ and $Q^2$ have the meaning indicated in claim 1 and Z is a group of the formula $-NHCH_2-$, $-NHCH(CH_3)-$, $-NHCH(C_6H_5)-$, -NHCH(COO-isobutyl)-, -NHCH(CO-Val)-, -NHCH(CO-Phe)-, -NHCH(CO-Tyr)-, $-NHCH(CO-Ser-OC_2H_5)-$, -NHCH(CO-Leu-O-isopropyl)-, $-NHCH(CONHCH_2CH_2-C_6H_4-OCH_3)$, $-NHCH(CONHCH_2CH_2C_6H_4-COOH)-$, $-NHCH(CONHCH_2CH_2-C_6H_4-OCH_2COOH)-$, $-NHCH(CONH_2)-$ or NHCH(pyrrolidinoyl)-.

**5.** A process according to claim 2, characterized in that compounds from the following group are prepared:

N-[N-[4-(p-Amidinobenzamido)butyryl]-L-α-aspartyl]-L-valine,
N-[N-(p-amidinobenzoyl)-β-alanyl]-β alanine and in particular
N-[N-[N-(p-amidinobenzoyl)-β-alanyl]-L-α-aspartyl]-3-phenyl-L-alanine.

**6.** A process according to claim 2, characterized in that compounds from the following group are prepared:

N-[N-[N-( 1-Amidino-4-piperidinylcarbonyl)-β-alanyl]-L-α-aspartyl]-3-phenyl-L-alanine,
N-[N-[N-(p-amidinophenylacetyl)-β-alanyl]-L-α-aspartyl]-3-phenyl-L-alanine,
N-[N-[4-(p-amidinophenylcarbamoyl)butyryl]-L-α-aspartyl]-3-phenyl-L-alanine,
N-[N-[(p-amidinophenylcarbamoyl)acetyl]-L-α-aspartyl]-3-phenyl-L-alanine,
rac-N-[1-(p-amidinobenzoyl)-3-piperidinylcarbonyl]-β-alanine,
N-[4-(p-amidinobenzamido)butyryl]-β-alanine and
N-[(DL)-N-(p-amidinobenzoyl)-3-phenyl-β-alanyl]-β-alanine .

**7.** A process according to claim 1, characterized in that compounds from the following group are prepared:

N-[N-[N-(p-Amidinobenzoyl)-β-alanyl]-L-α-aspartyl]-L-leucine isopropyl ester,
N-[N-[N-(p-amidinobenzoyl)-β-alanyl]-L-α-aspartyl]-L-valine,
N-[N-[N-(p-amidinobenzoyl)-β-alanyl]-L-α-aspartyl]-3-(p-hydroxyphenyl)-L-alanine,
N-[N-[5-(p-amidinobenzamido)valeryl]-L-α-aspartyl] -3-phenyl-L-alanine,
isobutyl N-[5-(p-amidinobenzamido)valeryl]-L-α-aspartate,
N-[N-[N-(p-amidinobenzoyl)-β-alanyl]-L-α-aspartyl-L-serine ethyl ester and
N-[N-[[(R)-1-(p-amidinobenzoyl)-3-pyrrolidinyl] carbonyl]-L-α-aspartyl]-3-phenyl-L-alanine.

**8.** A process according to claim 1, characterized in that compounds from the following group are prepared:

N-N'-[[(S)-(p-Amidinobenzamido)ethylene] dicarbonyl] di-β-alanine, N2-(p-amidinobenzoyl)-N4-(2-carboxyethyl)-L-aspargine,
N-[5-(p-amidinobenzamido)valeryl]-β-alanine,
rac-N-[[1-[3-(1-amidino-4-piperidinyl)propionyl]-3-piperidinyl]carbonyl-β-alanine,
N-[[(S)-1-(p-amidinobenzoyl-2-pyrrolidinyl]acetyl]-β-alanine,
(S)-3-[[N-(p-amidinobenzoyl)-β-alanyl]amino-3-[(p-methoxyphenethyl)carbamoyl]propionic acid,
N-[[(R)-1-(p-amidinobenzoyl)-3-pyrrolidinyl]carbonyl]-β-alanine,
N-[N-(p-amidinobenzoyl)-2-methyl-β-alanyl]-L-α-aspartamide,
N-[N-[(p-amidinophenyl)acetyl]-β-alanyl]-β-alanine,
rac-N-[[[1-(p-amidinophenyl)acetyl]-3-piperidinyl]carbonyl]-β-alanine benzyl ester,
rac-N-[[1[(p-amidinophenyl)acetyl]-3-piperidinyl]carbonyl]-β-alanine,
N-[N-[N-[3-(1-amidino-4-piperidinyl)propionyl]-β-alanyl]-L-α-aspartyl]-3-phenyl-L-alanine,
(S)-β-[[DL-N-(p-amidinobenzoyl)-3-methyl-β-alanyl]amino]-γ-oxo-1-pyrrolidinebutyric acid.
DL-N-[N-(p-amidinobenzoyl)-β-alanyl]-3-methyl-β-alanine,
N-[DL-N-(p-amidinobenzoyl)-2-phenyl-β-alanyl]-β-alanine,
DL-N-[N-(p-amidinobenzoyl)-β-alanyl]-2-methyl-β-alanine,
DL-N-[N-(p-amidinobenzoyl)-β-alanyl]-2-phenyl-β-alanine,
p-[2-[[N-[N-(p-amidinobenzoyl)-β-alanyl]-L-α-aspartyl]amino]ethyl]-benzoic acid,
DL-N-[N-(p-amidinobenzoyl)-β-alanyl-3-phenyl-β-alanine,
[p-[2-[[N-[N-(p-amidinobenzoyl)-β-alanyl]-L-α-aspartyl]amino]ethyl]phenoxy]acetic acid,
1-[N-(p-amidinobenzoyl)-β-alanyl]-4-piperidineacetic acid,

4-[N-(p-amidinobenzoyl)-β-alanyl]-1-piperazineacetic acid and
N-[N-[N-[(5-amidino-2-pyridyl)carbonyl]-β-alanyl]-L-α-aspartyl]-3-phenyl-L-alanine.

**9.** A process for the production of pharmaceutical preparations, in particular for the treatment of prophylaxis of diseases which are caused by the binding of adhesive proteins to blood platelet, and also by blood platelet aggregation and cell-cell adhesion, characterized by bringing a compound according to claim 1 or 2 into a galenical administration form.

**10.** The use of a compound according to claim 1 or 2 for the production of medicaments for the treatment or prophylaxis of diseases which are caused by the binding of adhesive proteins to blood platelets, and also by blood platelet aggregation and cell-cell adhesion, in particular for the treatment or prophylaxis of blood platelet thrombi, thrombosis, cerebral infarct, myocardial infarct, inflammation or arteriosclerosis, or as antitumour agents or as agents for the healing of wounds.

**11.** Acetic acid derivatives of the formula

$$X'\text{-}Y\text{-}CO\text{-}Z\text{-}CH(Q^1)\text{-}COOQ^2 \qquad II'$$

in which X' is phenyl or 4-piperidinyl substituted in the 4-position by an optionally protected amidino group and Y, Z, $Q^1$ and $Q^2$ have the same meaning as in claim 1, with the molecule containing at least one easily cleavable ester group or protected amidino group.

## Revendications

**Revendications pour les Etats contractants suivants : AT, BE, CH, DE, DK, FR, GB, IT, LI, LU, NL, SE**

**1.** Dérivés de l'acide acétique, contenant des liaisons peptidiques et répondant à la formule

$$H_2N(NH)C\text{-}X\text{-}Y\text{-}CO\text{-}Z\text{-}CH(Q^1)COOQ^2$$

dans laquelle

$Q^1$ représente l'hydrogène, un groupe méthyle ou phényle,
$Q^2$ représente l'hydrogène, un groupe phénylalkyle inférieur ou un groupe alkyle inférieur scindable dans l'organisme,
X représente un groupe 1,4-phénylène, 2,5- ou 3,6-pyridylène ou un groupe 1,4-pipéridinylène relié au groupe Y par l'intermédiaire de l'atome de carbone en position 4,
Y représente un groupe de formule

$$-(CH_2)_{0\text{-}2}\text{-}CONHCH(Q^3)(CH_2)_{1\text{-}3}\text{-} \qquad (Y^1)$$

$$-CONHCH_2CH(Q^4)\text{-} \qquad (Y^2)$$

$$-(CH_2)_2NHCOCH_2\text{-} \qquad (Y^3)$$

$$-NHCO(CH_2)_3\text{-} \qquad (Y^4)$$

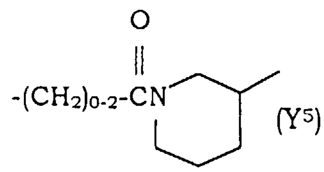

(Y5)

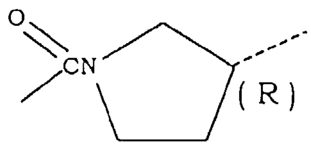

(Y6)

ou

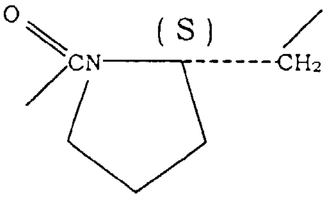

(Y7)

| | | |
|---|---|---|
| $Q^3$ | représente l'hydrogène, un groupe méthyle, phényle, -COOH ou -CONH$(CH_2)_2$-COOH, |
| $Q^4$ | représente l'hydrogène, un groupe méthyle ou phényle, |
| Z | représente un groupe 1,4-pipérazinylène, un groupe 1,4-pyridinylène relié au groupe CO par l'atome d'azote en position 1, ou un groupe de formule |

$$-NHCH(R^1)-$$

ou

$$NHCH(COR^2)-$$

| | |
|---|---|
| $R^1$ | représente l'hydrogène, un groupe méthyle, phényle ou -COO-alkyle inférieur, |
| $R^2$ | représente le radical d'un acide α-aminocarboxylique relié par le groupe amino ou d'un ester de cet acide, ou bien un groupe de formule -$NHCH_2CH_2$-Ar, ou bien |
| -CO-$R^2$ | représente un groupe carbamoyle ou un groupe pyrrolidinoyle ou pipéridinoyle, |
| Ar | représente un groupe phényle ou un groupe phényle substitué par un groupe alkyle inférieur, alcoxy inférieur, -COOH, -COO-alkyle inférieur, -O$(CH_2)_{1-4}$-COOH ou O$(CH_2)_{1-4}$-COO-alkyle inférieur, |

leurs hydrates ou solvates et leurs sels acceptables pour l'usage pharmaceutique.

**2.** Composés selon la revendication 1, de formule

$$H_2N(HN)C-X-Y^a-CONHCH(R^{11})CH_2COOQ^{21} \qquad \text{I-A}$$

dans laquelle

| | |
|---|---|
| X | représente un groupe 1,4-phénylène ou un groupe 1,4-pipéridinylène relié au groupe $Y^a$ par l'intermédiaire de l'atome de carbone en position 4, |
| $Y^a$ | représente un groupe de formule |

$-(CH_2)_{0-1}-CONHCH(Q^a)(CH_2)_{1-2}-$ ($Y^{11}$)

$-(CH_2)_2NHCOCH_2-$ ($Y^3$)

$-NHCO(CH_2)_3-$ ($Y^4$)

ou

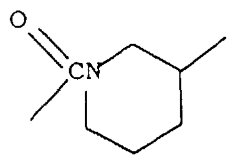

($Y^{51}$)

$Q^a$ représente l'hydrogène ou un groupe phényle,
$R^{11}$ représente l'hydrogène ou un groupe $-CO-R^{22}$,
$R^{22}$ représente le radical d'un acide α-aminocarboxylique relié par le groupe amino ou d'un ester d'un tel acide,
$Q^{21}$ représente l'hydrogène ou un groupe alkyle inférieur scindable dans l'organisme, ainsi que les hydrates ou solvates et sels acceptables pour l'usage pharmaceutique de ces composés.

**3.** Composés selon la revendication 1, pour lesquels X, Z, $Q^1$ et $Q^2$ ont les significations indiquées dans la revendication 1, et Y représente un groupe de formule $Y^1$ en particulier un groupe de formule :

$-CONH(CH_2)_{2-4}-$,

$-CH_2CONH(CH_2)_2-$,

$-CONHCH(C_6H_5)CH_2-$,

$-CONHCH(CONHCH_2CH_2COOH)CH_2-$,

$-CONHCH(COOH)CH_2-$,

ou

$-CONHCH(CH_3)CH_2-$.

**4.** Composés selon la revendication 1, pour lesquels X, Y, $Q^1$ et $Q^2$ ont les significations indiquées dans la revendication 1, et Z représente un groupe de formule $-NHCH_2-$, $-NHCH(CH_3)-$, $-NHCH(C_6H_5)-$, -NHCH(COO-isobutyl)-, -NHCH(CO-Val)-, NHCH(CO-Phe)-, NHCH(CO-Tyr)-, $NHCH(CO-Ser-OC_2H_5)-$, NHCH(CO-Leu-O-isopropyl)-, $NHCH(CONHCH_2CH_2-C_6H_4-OCH_3)-$, $-NHCH\ (CONHCH_2CH_2-C_6H_4-COOH)-$, $-NHCH(CONHCH_2CH_2-C_6H_4-OCH_2COOH)-$, $-NHCH(CONH_2)-$ ou -NHCH(pyrrolidinoyl)-.

**5.** Composés selon la revendication 2, pris dans le groupe suivant

- ☐ N-[N-[4-(p-amidinobenzamido)butyryl]-L-α-aspartyl]-L-valine,
- ☐ N-[N-(p-amidinobenzoyl)-β-alanyl]-β-alanine et spécialement
- ☐ N-[N-[N-(p-amidinobenzoyl)-β-alanyl]-L-α-aspartyl]-3-phényl-L-alanine.

**6.** Composés selon la revendication 2, pris dans le groupe suivant :

- ☐ N-[N-[N-(1-amidino-4-piperidinylcarbonyl)-β-alanyl]-L-α-aspartyl]-3-phényl-L-alanine
- ☐ N-[N-[N-(p-amidinophénylacétyl)-β-alanyl]-L-α-aspartyl]-3-phényl-L-alanine
- ☐ N-[N-[4-(p-amidinophénylcarbamoyl)-butyryl]-L-α-aspartyl]-3-phényl-L-alanine
- ☐ N-[N-[(p-amidinophénylcarbamoyl)-acétyl]-L-α-aspartyl]-3-phényl-L-alanine
- ☐ rac-N-[1-(p-amidinobenzoyl)-3-pipéridinylcarbonyl]-β-alanine
- ☐ N-[4-(p-amidinobenzamido)-butyryl]-β-alanine et
- ☐ N-(DL)-N-(p-amidinobenzoyl)-3-phényl-β-alanyl]-β-alanine.

**7.** Composés selon revendication 1, pris dans le groupe suivant :

- ☐ Ester isopropylique de la N-[N-[N-(p-amidinobenzoyl)-β-alanyl]-L-α-aspartyl]-L-leucine,
- ☐ N-[N-[N-(p-amidinobenzoyl)-β-alanyl]-L-α-aspartyl]-L-valine,
- ☐ N-[N-[N-(p-amidinobenzoyl)-β-alanyl]-L-α-aspartyl]-3-(p-hydroxyphényl)-L-alanine,
- ☐ N-[N-[5-(p-amidinobenzamido)valéryl]-L-α-aspartyl]-3-phényl-L-alanine,
- ☐ N-[5-(p-amidinobenzamido)-valéryl]-L-α-aspartate d'isobutyle,
- ☐ Ester éthylique de la N-[N-[N-(p-amidinobenzoyl)-β-alanyl]-L-α-aspartyl]-L-sérine et
- ☐ N-[N-[[(R)-1-(p-amidinobenzoyl)-3-pyrrolidinyl]-carbonyl]-L-α-aspartyl]-3-phényl-L-alanine.

**8.** Composés selon la revendication 1, pris dans le groupe suivant :

- ☐ N,N'-[[(S)-(p-amidinobenzamido)-éthylène]-dicarbonyl]-di-β-alanine,
- ☐ N2-(p-amidinobenzoyl)-N4-(2-carboxyéthyl)-L-asparagine,
- ☐ N-[5-(p-amidinobenzamido)-valéryl]-β-alanine,
- ☐ rac-N-[[1-[3-(1-amidino-4-pipéridinyl)-propionyl]-3-pipéridinyl]carbonyl]-β-alanine,
- ☐ N-[[(S)-1-(p-amidinobenzoyl)-2-pyrrolidinyl]-acétyl]-β-alanine,
- ☐ Acide (S)-3-[[N-(p-amidinobenzoyl)-β-alanyl]-amino-3-[(p-méthoxyphénéthyl)-carbamoyl]-propionique,
- ☐ N-[[(R)-1-(p-amidinobenzoyl)-3-pyrrolidinyl]-carbonyl]-β-alanine,
- ☐ α-amide de l'acide N-[N-(p-amidinobenzoyl)-2-méthyl-β-alanyl]-L-aspartique,
- ☐ N-[N-[(p-amidinophényl)-acétyl]-β-alanyl]-β-alanine,
- ☐ Ester benzylique de la rac-N-[[[1-(p-amidinophényl)-acétyl]-3-pipéridinyl]-carbonyl]-β-alanine,
- ☐ rac-N-[[1-[(p-amidinophényl)-acétyl]-3-pipéridinyl)-carbonyl]-β-alanine,
- ☐ N-[N-[N-[3-(1-amidino-4-pipéridinyl)-propionyl]-β-alanyl]-L-α-aspartyl]-3-phényl-L-alanine,
- ☐ Acide (S)-β-[[DL-N-(p-amidinobenzoyl)-3-méthyl-β-alanyl]-amino-γ-oxo-1-pyrrolidinbutyrique,
- ☐ DL-N-[N-(p-amidinobenzoyl)-β-alanyl]-3-méthyl-β-alanine,
- ☐ N-[DL-N-(p-amidinobenzoyl)-2-phényl-β-alanyl]-β-alanine,
- ☐ DL-N-[N-(p-amidinobenzoyl)-β-alanyl]-2-méthyl-β-alanine,
- ☐ DL-N-[N-(p-amidinobenzoyl)-β-alanyl]-2-phényl-β-alanine,
- ☐ Acide p-[2-[[N-[N-(p-amidinobenzoyl)-β-alanyl]-L-α-aspartyl]-amino]-éthyl]-benzoique,
- ☐ DL-N-[N-(p-amidinobenzoyl)-β-alanyl-3-phényl-β-alanine,
- ☐ Acide [p-[2-[[N-[N-(p-amidinobenzoyl)-β-alanyl]-L-α-aspartyl]-amino]-éthyl]-phénoxy]acétique,
- ☐ Acide 1-[N-(p-amidinobenzoyl)-β-alanyl]-4-piperidinacétique,
- ☐ Acide 4-[N-(p-amidinobenzoyl)-β-alanyl]-1-pipérazinacétique et
- ☐ N-[N-[N-[(5-amidino-2-pyridyl)-carbonyl]-β-alanyl]-L-α-aspartyl]-3-phényl-L-alanine.

**9.** Dérivés de l'acide acétique de formule

$$X'\text{-}Y\text{-}CO\text{-}Z\text{-}CH(Q^1)\text{-}COOQ^2$$

dans laquelle X' représente un groupe phényle ou 4-pipéridinyle substitué en position 4 par un groupe amidino éventuellement protégé, et Y, Z, $Q^1$ et $Q^2$ ont les significations indiquées dans la revendication 1, la molécule contenant au moins un groupe ester ou un groupe amidino protégé, facile à scinder.

**10.** Composés selon l'une des revendications 1 à 8, pour l'utilisation en tant que substances actives pharmaceutiques.

**11.** Procédé de préparation des composés selon la revendication 1, caractérisé en ce que

a) dans un composé de formule

$$X'\text{-}Y\text{-}CO\text{-}Z'\text{-}CH(Q^1)\text{-}COO\text{-}Q^5 \qquad II$$

dans laquelle

- $Q^1$ et Y — ont les significations indiquées dans la revendication 1,
- X' — représente un groupe phényle ou 4-pipéridinyle substitué en position 4 par un groupe amidino éventuellement protégé, et
- Z' et $Q^5$ — ont les significations indiquées dans la revendication 1 pour les symboles Z et $Q^2$

on scinde au moins un groupe protecteur, ou bien
b) dans un nitrile de formule

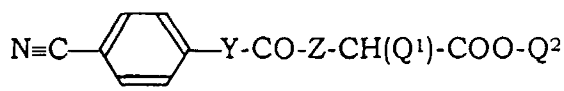

III

on convertit le groupe nitrile en groupe amidino et, si on le désire, on convertit un composé de formule I en un sel acceptable pour l'usage pharmaceutique ou bien on convertit un sel d'un composé de formule I en l'acide ou la base libre.

**12.** Compositions pharmaceutiques, convenant en particulier pour le traitement ou la prophylaxie de maladies causées par la fixation de protéines adhésives sur les plaquettes du sang ou par l'agglomération des plaquettes du sang et l'adhérence cellule-cellule, qui contiennent un composé selon la revendication 1 ou 2 en tant que substance active.

**13.** Utilisation d'un composé selon la revendication 1 ou 2 pour la préparation de médicaments servant au traitement ou à la prophylaxie de maladies causées par la fixation de protéines adhésives sur les plaquettes du sang ou par l'agglomération des plaquettes du sang et l'adhérence cellule-cellule, et en particulier pour le traitement ou la prophylaxie des caillots de plaquettes du sang, des thromboses, de la congestion cérébrale, de l'infarctus cardiaque, des inflammations ou de l'arteriosclérose, ou en tant qu'agents anti-tumeurs ou médicaments pour la cicatrisation des blessures.

**Revendications pour les Etats contractants suivants : ES, GR**

**1.** Procédé de préparation des dérivés de l'acide acétique, contenant des liaisons peptidiques, qui répondent à la formule

$$H_2N(NH)C\text{-}X\text{-}Y\text{-}CO\text{-}Z\text{-}CH(Q^1)COOQ^2 \qquad I$$

dans laquelle

- $Q^1$ — représente l'hydrogène, un groupe méthyle ou phényle,
- $Q^2$ — représente l'hydrogène, un groupe phénylalkyle inférieur ou un groupe alkyle inférieur scindable dans l'organisme,
- X — représente un groupe 1,4-phénylène, 2,5- ou 3,6-pyridylène ou un groupe 1,4-pipéridinylène relié au groupe Y par l'intermédiaire de l'atome de carbone en position 4,
- Y — représente un groupe de formule

$$-(CH_2)_{0\text{-}2}\text{-}CONHCH(Q^3)(CH_2)_{1\text{-}3}\text{-} \qquad (Y^1)$$

$-CONHCH_2CH(Q^4)-$ ($Y^2$)

$-(CH_2)_2NHCOCH_2-$ ($Y^3$)

$-NHCO(CH_2)_3-$ ($Y^4$)

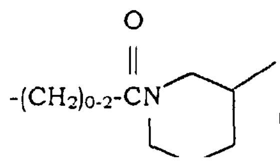

($Y^5$)

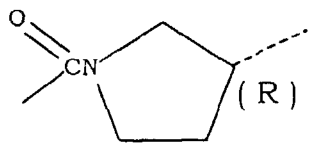

($Y^6$)

ou

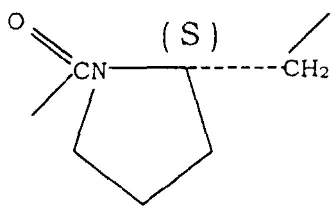

($Y^7$)

- $Q^3$ représente l'hydrogène, un groupe méthyle, phényle, -COOH ou $-CONH(CH_2)_2-COOH$,
- $Q^4$ représente l'hydrogène, un groupe méthyle ou phényle,
- Z représente un groupe 1,4-pipérazinyle, un groupe 1,4-pipéridinyle relié au groupe CO par l'atome d'azote en position 1, ou un groupe de formule

$$-NHCH(R^1)-$$

ou

$$-NHCH(COR^2)-$$

- $R^1$ représente l'hydrogène, un groupe méthyle, phényle ou -COO-alkyle inférieur,
- $R^2$ représente le radical d'un acide α-aminocarboxylique relié par l'intermédiaire du groupe amino ou d'un ester d'un tel acide, ou un groupe de formule $-NHCH_2CH_2-Ar$, ou bien
- $-CO-R^2$ représente un groupe carbamoyle ou un groupe pyrrolidinoyle ou pipéridinoyle,
- Ar représente un groupe phényle ou un groupe phényle substitué par un groupe alkyle inférieur, alcoxy inférieur, -COOH, -COO-alkyle inférieur, $-O(CH_2)_{1-4}-COOH$ ou $O(CH_2)_{1-4}-COO$-alkyle inférieur,

de leurs hydrates ou solvates et de leurs sels acceptables pour l'usage pharmaceutique, caractérisé en ce que

a) dans un composé de formule

$$X'\text{-}Y\text{-}CO\text{-}Z'\text{-}CH(Q^1)\text{-}COO\text{-}Q^5 \qquad II$$

dans laquelle

- $Q^1$ et Y ont les significations indiquées dans la revendication 1,
- X' représente un groupe phényle ou un groupe 4-pipéridinyle substitué en position 4 par un groupe amidino éventuellement protégé, et
- Z' et $Q^5$ ont respectivement les significations indiquées ci-dessus pour Z et $Q^2$

on élimine au moins un groupe protecteur, ou bien
b) dans un nitrile de formule

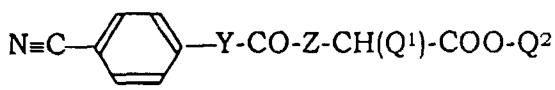

III

on convertit le groupe nitrile en groupe amidino, et si on le désire, on convertit un composé de formule I en un sel acceptable pour l'usage pharmaceutique ou bien on convertit un sel d'un composé de formule I en l'acide ou la base libre.

**2.** Procédé selon la revendication 1, caractérisé en ce que l'on prépare des composés de formule

$$H_2N(HN)C\text{-}X\text{-}Y^a\text{-}CONHCH(R^{11})CH_2COOQ^{21} \qquad I\text{-}A$$

dans laquelle

- X représente un groupe 1,4-phénylène ou un groupe 1,4-pipéridinylène relié au groupe $Y^a$ par l'atome de carbone en position 4,
- $Y^a$ représente un groupe de formule

$$-(CH_2)_{0\text{-}1}\text{-}CONHCH(Q^a)(CH_2)_{1\text{-}2}\text{-} \qquad (Y^{11})$$

$$-(CH_2)_2NHCOCH_2\text{-} \qquad (Y^3)$$

$$-NHCO(CH_2)_3\text{-} \qquad (Y^4)$$

ou

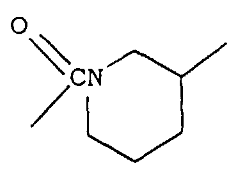

($Y^{51}$)

- $Q^a$ représente l'hydrogène ou un groupe phényle,
- $R^{11}$ représente l'hydrogène ou un groupe -CO-$R^{22}$,
- $R^{22}$ représente le radical d'un acide α-aminocarboxylique relié par le groupe amino ou d'un ester d'un tel acide,

$Q^{21}$ représente l'hydrogène ou un groupe alkyle inférieur scindable dans l'organisme, ou les hydrates ou solvates et sels acceptables pour l'usage pharmaceutique de ces composés.

**3.** Procédé selon la revendication 1, dans lequel X, Z, $Q^1$ et $Q^2$ ont les significations indiquées dans la revendication 1, et Y représente un groupe de formule $Y^1$ en particulier un groupe de formule

$-CONH(CH_2)_{2-4}-$,

$-CH_2CONH(CH_2)_2-$,

$-CONHCH(C_6H_5)CH_2-$,

$-CONHCH(CONHCH_2CH_2COOH)CH_2-$,

$-CONHCH(COOH)CH_2-$,

ou

$-CONHCH(CH_3)CH_2-$.

**4.** Procédé selon la revendication 1, dans lequel X, Y, $Q^1$ et $Q^2$ ont les significations indiquées dans la revendication 1, et Z représente un groupe de formule $-NHCH_2-$, $-NHCH(CH_3)-$, $-NHCH(C_6H_5)-$, -NHCH(COO-isobutyl)-, -NHCH (CO-Val)-, NHCH(CO-Phe)-, NHCH(CO-Tyr)-, NHCH(CO-Ser-$OC_2H_5$)-, NHCH(CO-Leu-O-isopropyl)-, NHCH ($CONHCH_2CH_2$-$C_6H_4$-$OCH_3$)-, -NHCH ($CONHCH_2CH_2$-$C_6H_4$-COOH)-, -NHCH($CONHCH_2CH_2$-$C_6H_4$-$OCH_2COOH$)-, -NHCH($CONH_2$)- ou -NHCH(pyrrolidinoyl)-,

**5.** Procédé selon la revendication 2, caractérisé en ce que l'on prépare des composés du groupe suivant:

- ☐ N-[N-[4-(p-amidinobenzamido)butyryl]-L-α-aspartyl]-L-valine,
- ☐ N-[N-(p-amidinobenzoyl)-β-alanyl]-β-alanine et spécialement
- ☐ N-[N-[N-(p-amidinobenzoyl)-β-alanyl]-L-α-aspartyl]-3-phényl-L-alanine.

**6.** Procédé selon la revendication 2, caractérisé en ce que l'on prépare des composés du groupe suivant :

- ☐ N-[N-[N-(1-amidino-4-pipéridinylcarbonyl)-β-alanyl]-L-α-aspartyl]-3-phényl-L-alanine
- ☐ N-[N-[N-(p-amidinophénylacétyl)-β-alanyl]-L-α-aspartyl]-3-phényl-L-alanine
- ☐ N-[N-[4-(p-amidinophénylcarbamoyl)-butyryl]-L-α-aspartyl]-3-phényl-L-alanine
- ☐ N-[N-[(p-amidinophénylcarbamoyl)-acétyl]-L-α-aspartyl]-3-phényl-L-alanine
- ☐ rac-N-[1-(p-amidinobenzoyl)-3-pipéridinylcarbonyl]-β-alanine
- ☐ N-[4-(p-amidinobenzamido)-butyryl]-β-alanine et
- ☐ N-(DL)-N-(p-amidinobenzoyl)-3-phényl-β-alanyl]-2-alanine.

**7.** Procédé selon revendication 1, caractérisé en ce que l'on prépare des composés du groupe suivant :

- ☐ Ester isopropylique de la N-[N-[N-(p-amidinobenzoyl)-β-alanyl]-L-α-aspartyl]-L-leucine,
- ☐ N-[N-[N-(p-amidinobenzoyl)-β-alanyl]-L-α-aspartyl]-L-valine,
- ☐ N-[N-[N-(p-amidinobenzoyl)-β-alanyl]-L-α-aspartyl]-3-(p-hydroxyphényl)-L-alanine,
- ☐ N-[N-[5-(p-amidinobenzamido)-valéryl]-L-α-aspartyl]-3-phényl-L-alanine,
- ☐ N-[5-(p-amidinobenzamido)-valéryl]-L-α-aspartate d'isobutyle,
- ☐ Ester éthylique de la N-[N-[N-(p-amidinobenzoyl)-β-alanyl]-L-α-aspartyl]-L-sérine et
- ☐ N-[N-[[(R)-1-(p-amidinobenzoyl)-3-pyrrolidinyl]-carbonyl]-L-α-aspartyl]-3-phényl-L-alanine.

**8.** Procédé selon la revendication 1, caractérisé en ce que l'on prépare des composés du groupe suivant :

- ☐ N,N'-[[(S)-(p-amidinobenzamido)-éthylène]-dicarbonyl]-di-β-alanine,
- ☐ N2-(p-amidinobenzoyl)-N4-(2-carboxyéthyl)-L-asparagine,
- ☐ N-[5-(p-amidinobenzamido)-valéryl]-β-alanine,
- ☐ rac-N-[[1-[3-(1-amidino-4-pipéridinyl)-propionyl]-3-pipéridinyl]-carbonyl]-β-alanine,
- ☐ N-[[(S)-1-(p-amidinobenzoyl)-2-pyrrolidinyl]-acétyl]-β-alanine,
- ☐ Acide (S)-3-[[N-(p-amidinobenzoyl)-β-alanyl]-amino-3-[(p-méthoxyphénéthyl)-carbamoyl]-propionique,
- ☐ N-[[(R)-1-(p-amidinobenzoyl)-3-pyrrolidinyl]-carbonyl]-β-alanine,
- ☐ α-amide de l'acide N-[N-(p-amidinobenzoyl)-2-méthyl-β-alanyl]-L-aspartique,
- ☐ N-[N-[(p-amidinophényl)-acétyl]-β-alanyl]-β-alanine,
- ☐ Ester benzylique de la rac-N-[[[1-(p-amidinophényl)-acétyl]-3-pipéridinyl]-carbonyl]-β-alanine,
- ☐ rac-N-[[1-[(p-amidinophényl)-acétyl]-3-pipéridinyl)-carbonyl]-β-alanine,
- ☐ N-[N-[N-[3-(1-amidino-4-pipéridinyl)-propionyl]-β-alanyl]-L-α-aspartyl]-3-phényl-L-alanine,
- ☐ Acide (S)-β[[DL-N-(p-amidinobenzoyl)-3-méthyl-β-alanyl]-amino]-γ-oxo-1-pyrrolidinbutyrique,
- ☐ DL-N-[N-(p-amidinobenzoyl)-β-alanyl]-3-méthyl-β-alanine,
- ☐ N-[DL-N-(p-amidinobenzoyl)-2-phényl-β-alanyl]-β-alanine,
- ☐ DL-N-[N-(p-amidinobenzoyl)-β-alanyl]-2-méthyl-β-alanine,
- ☐ DL-N-[N-(p-amidinobenzoyl)-β-alanyl]-2-phényl-β-alanine,
- ☐ Acide p-[2-[[N-[N-(p-amidinobenzoyl)-β-alanyl]-L-α-aspartyl]-amino]-éthyl]-benzoïque,
- ☐ DL-N-[N-(p-amidinobenzoyl)-β-alanyl-3-phényl-β-alanine,
- ☐ Acide [p-[2-[[N-[N-(p-amidinobenzoyl)-β-alanyl]-L-α-aspartyl]-amino]-éthyl]-phénoxy]acétique,
- ☐ Acide 1-[N-(p-amidinobenzoyl)-β-alanyl]-4-pipéridinacétique,
- ☐ Acide 4-[N-(p-amidinobenzoyl)-β-alanyl]-1-pipérazinacétique et N-[N-[N-[(5-amidino-2-pyridyl)-carbonyl]-β-alanyl]-L-α-aspartyl]-3-phényl-L-alanine.

**9.** Procédé de préparation de compositions pharmaceutiques prévues en particulier pour le traitement ou la prophylaxie de maladies dues à la fixation de protéines adhésives sur les plaquettes du sang ou par une agglomération des plaquettes du sang et l'adhérence cellule-cellule, caractérisé en ce que l'on met un composé selon la revendication 1 ou 2 sous une forme d'administration galénique.

**10.** Utilisation d'un composé selon la revendication 1 ou 2 pour la préparation de médicaments servant au traitement ou à la prophylaxie de maladies causées par la fixation de protéines adhésives sur les plaquettes du sang ou par l'agglomération des plaquettes du sang et l'adhérence cellule-cellule, et en particulier pour le traitement ou la prophylaxie des caillots de plaquettes du sang, des thromboses, de la congestion cérébrale, de l'infarctus cardiaque, des inflammations ou de l'arteriosclérose, ou en tant qu'agents anti-tumeurs ou médicaments pour la cicatrisation des blessures.

**11.** Dérivés de l'acide acétique de formule

$$X'\text{-}Y\text{-}CO\text{-}Z\text{-}CH(Q^1)\text{-}COOQ^2$$

dans laquelle X' représente un groupe phényle ou 4-pipéridinyle substitué en position 4 par un groupe amidino éventuellement protégé, et Y, Z, $Q^1$ et $Q^2$ ont les significations indiquées dans la revendication 1, la molécule contenant au moins un groupe ester ou un groupe amidino protégé, facile à scinder.